(19) Europäisches Patentamt
European Patent Office
Office européen des brevets

(11) Veröffentlichungsnummer: **0 425 948 B1**

# (12) EUROPÄISCHE PATENTSCHRIFT

(45) Veröffentlichungstag der Patentschrift: **13.12.95**

(21) Anmeldenummer: **90120152.5**

(22) Anmeldetag: **20.10.90**

Teilanmeldung 95101833.2 eingereicht am 20/10/90.

Die Akte enthält technische Angaben, die nach dem Eingang der Anmeldung eingereicht wurden und die nicht in dieser Patentschrift enthalten sind.

(51) Int. Cl.[6]: **C07D 249/12**, C07D 401/14, C07D 403/12, C07D 401/12, C07D 409/12, C07D 417/12, A01N 47/38

## (54) Halogenierte Sulfonylaminocarbonyltriazolinone

(30) Priorität: **03.11.89 DE 3936622**

(43) Veröffentlichungstag der Anmeldung: **08.05.91 Patentblatt 91/19**

(45) Bekanntmachung des Hinweises auf die Patenterteilung: **13.12.95 Patentblatt 95/50**

(84) Benannte Vertragsstaaten: **BE CH DE DK ES FR GB IT LI NL**

(56) Entgegenhaltungen:
**EP-A- 0 283 876**
**EP-A- 0 341 489**

(73) Patentinhaber: **BAYER AG**
**D-51368 Leverkusen (DE)**

(72) Erfinder: **Müller, Klaus-Helmut, Dr.**
**Bockhackstrasse 55**
**W-4000 Düsseldorf 13 (DE)**
Erfinder: **Babczinski, Peter, Dr.**
**In der Lohrenbeck 11**
**W-5600 Wuppertal 1 (DE)**
Erfinder: **Santel, Hans-Joachim, Dr.**
**Grünstrasse 9a**
**W-5090 Leverkusen 1 (DE)**
Erfinder: **Schmidt, Robert R., Dr.**
**Im Waldwinkel 110**
**W-5060 Bergisch Gladbach 2 (DE)**

**EP 0 425 948 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue halogenierte Sulfonylaminocarbonyltriazolinone, mehrere Verfahren zu ihrer Herstellung und ihre Verwendung als Herbizide.

Es ist bekannt, daß bestimmte substituierte Aminocarbonylimidazolidinone, wie z. B. 1-Isobutylaminocarbonyl-2-imidazolidinon (Isocarbamid), herbizide Eigenschaften aufweisen (vgl. R. Wegler, Chemie der Pflanzenschutz- und Schädlingsbekämpfungsmittel, Band 5, S. 219, Springer-Verlag, Berlin-Heidelberg-New York, 1977).

Ferner ist bereits bekannt, daß bestimmte substituierte 2-Aminocarbonyl-5-(amino- oder mercapto)-2,4-dihydro-3H-1,2,4-triazol-3-one herbizid wirksam sind (EP-A-283 876).

Die Wirkung der vorbekannten Verbindungen ist jedoch nicht in allen Belangen zufriedenstellend.

Es wurden nun die neuen halogenierten Sulfonylaminocarbonyl-triazolinone der allgemeinen Formel (I) gefunden,

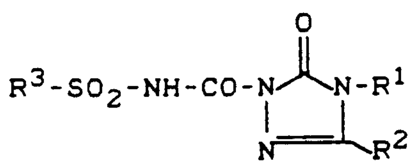

(I)

in welcher

$R^1$ für Wasserstoff, Hydroxy, Amino, für gegebenenfalls durch Fluor, Chlor, Brom, Cyano, $C_1$-$C_4$-Alkoxy, $C_1$-$C_4$-Alkylcarbonyl oder $C_1$-$C_4$-Alkoxy-carbonyl substituiertes $C_1$-$C_6$-Alkyl, für jeweils gegebenenfalls durch Fluor, Chlor und/oder Brom substituiertes $C_3$-$C_6$-Alkenyl oder $C_3$-$C_6$-Alkinyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio, $C_1$-$C_4$-Alkylsulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für $C_3$-$C_4$-Alkenyloxy, für gegebenenfalls durch Fluor, Cyano, Phenyl, $C_1$-$C_4$-Alkoxy oder $C_1$-$C_4$-Alkoxy-carbonyl substituiertes $C_1$-$C_4$-Alkylamino, $C_3$-$C_6$-Cycloalkylamino oder für Di-($C_1$-$C_4$-alkyl)-amino steht,

$R^2$ für Fluor, Chlor, Brom oder Jod steht und

$R^3$ für die Gruppierung

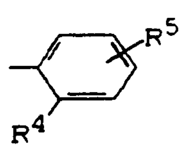

steht, worin

$R^4$ und $R^5$ gleich oder verschieden sind und für Wasserstoff, Fluor, Chlor, Brom, Iod, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor,

Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alxylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthiooder $C_1$-$C_4$-Alkoxycarbonyl substituiert ist), $C_3$-$C_6$-Alkinyloxy, $C_3$-$C_6$-Alkinylthio oder für den Rest -S-$(O)_p$-$R^6$ stehen, wobei

p für die Zahlen 1 oder 2 steht und

$R^6$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino, Phenyl oder für den Rest -NHOR$^7$ steht, wobei

$R^7$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^4$ und/oder $R^5$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest -CO-$R^8$ stehen, wobei

$R^8$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$ Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^4$ und/oder $R^5$ weiterhin für Trimethylsilyl, Thiazolinyl, $C_1$-$C_4$-Alkylsulfonyloxy, Di-($C_1$-$C_4$-alkyl)-aminosulfonylamino oder für den Rest -CH=N-$R^9$ stehen, wobei

$R^9$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiterhin

$R^3$ für den Rest

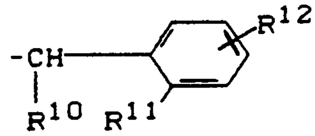

steht, worin

$R^{10}$ für Wasserstoff oder $C_1$-$C_4$-Alkyl steht,

$R^{11}$ und $R^{12}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-

Alkoxy (welches gegebenenfalls durch Fluor und/oder Chlor substituiert ist), Carboxy, $C_1$-$C_4$-Alkoxy-carbonyl, Dimethylaminocarbonyl, $C_1$-$C_4$-Alkylsulfonyl oder Di-($C_1$-$C_4$-alkyl)-aminosulfonyl stehen;

weiterhin

$R^3$ für den Rest

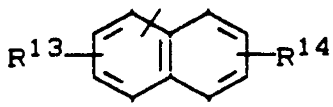

steht, worin

$R^{13}$ und $R^{14}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiterhin

$R^3$ für den Rest

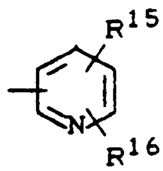

steht, worin

$R^{15}$ und $R^{16}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), für Aminosulfonyl, Mono-($C_1$-$C_4$-alkyl)-aminosulfonyl, Di-($C_1$-$C_4$- alkyl)-aminosulfonyl oder $C_1$-$C_4$-Alkoxy-carbonyl oder Dimethylaminocarbonyl stehen;

weiterhin

$R^3$ für den Rest

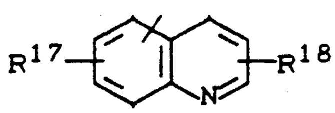

steht, worin

$R^{17}$ und $R^{18}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

weiterhin

$R^3$ für den Rest

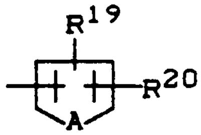

steht, worin

$R^{19}$ und $R^{20}$ gleich oder verschieden sind und für Wasserstoff, Fluor, Chlor, Brom, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl, $C_1$-$C_4$-Alkoxy-carbonyl oder Dimethylaminocarbonyl stehen, und

A für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiterhin

$R^3$ für den Rest

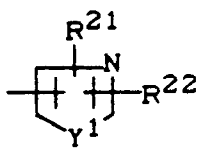

steht, worin

$R^{21}$ und $R^{22}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{23}$ steht, wobei

$R^{23}$ für Wasserstoff oder $C_1$-$C_4$-Alkyl steht;

weiterhin

$R^3$ für den Rest

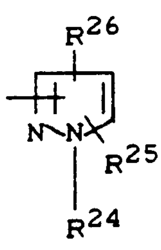

steht, worin

$R^{24}$ für Wasserstoff, $C_1$-$C_4$-Alkyl, Benzyl, Pyridyl, Chinolinyl oder Phenyl steht,

$R^{25}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{26}$ für Wasserstoff, Halogen oder $C_1$-$C_4$-Alkyl steht;

weiterhin

$R^3$ für eine der nachstehend aufgeführten Gruppierungen steht:

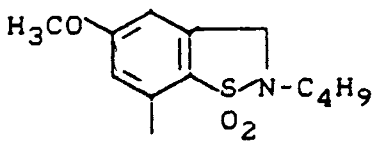 ; 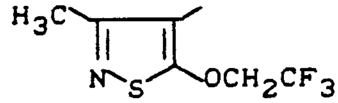

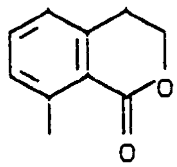

sowie Salze von Verbindungen der Formel (I).

Man erhält die neuen halogenierten Sulfonylaminocarbonyltriazolinone der allgemeinen Formel (I), wenn man

a) halogenierte Triazolinone der allgemeinen Formel (II)

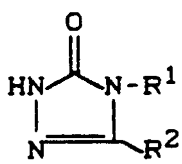 (II)

in welcher

$R^1$ und $R^2$ die oben angegebenen Bedeutungen haben,

mit Sulfonylisocyanaten der allgemeinen Formel (III)

$R^3$-$SO_2$-N = C = O (III)

in welcher

$R^3$ die oben angegebene Bedeutung hat,

gegebenenfalls in Gegenwart eines Verdünnungsmittels umsetzt, oder wenn man

b) halogenierte Triazolinon-Derivate der allgemeinen Formel (IV)

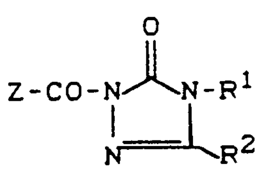 (IV)

in welcher

$R^1$ und $R^2$ die oben angegebenen Bedeutungen haben und

Z für Chlor, $C_1$-$C_4$-Alkoxy, Benzyloxy oder Phenoxy steht,

mit Sulfonsäureamiden der allgemeinen Formel (V)

$R^3$-$SO_2$-$NH_2$ (V)

in welcher

$R^3$ die oben angegebene Bedeutung hat,

gegebenenfalls in Gegenwart eines Säureakzeptors und gegebenenfalls in Gegenwart eines Verdünnungsmittels umsetzt, oder wenn man

c) halogenierte Triazolinone der allgemeinen Formel (II)

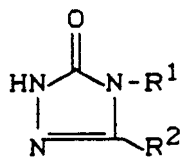

(II)

in welcher
$R^1$ und $R^2$ die oben angegebenen Bedeutungen haben,
mit Sulfonsäureamid-Derivaten der allgemeinen Formel (VI)

$R^3$-$SO_2$-NH-CO-Z (VI)

in welcher
$R^3$ die oben angegebene Bedeutung hat und
Z für Chlor, $C_1$-$C_4$-Alkoxy, Benzyloxy oder Phenoxy steht,
gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

Die neuen halogenierten Sulfonylaminocarbonyltriazolinone der allgemeinen Formel (I) und ihre Salze zeichnen sich durch starke herbizide Wirksamkeit aus.

Überraschenderweise zeigen die neuen Verbindungen der Formel (I) erheblich bessere herbizide Wirkung als das strukturell ähnliche bekannte Herbizid 1-Isobutylaminocarbonyl-2-imidazolidinon (Isocarbamid).

Gegenstand der Erfindung sind vorzugsweise Natrium-, Kalium-, Magnesium-, Calcium-, Ammonium-, $C_1$-$C_4$-Alkyl-ammonium-, Di-($C_1$-$C_4$-alkyl)-ammonium-, Tri-($C_1$-$C_4$-alkyl)-ammonium-, $C_5$- oder $C_6$-Cycloalkyl-ammonium- und Di-($C_1$-$C_2$-alkyl)-benzyl-ammonium-Salze von Verbindungen der Formel (I), in welcher $R^1$, $R^2$ und $R^3$ die oben angegebenen Bedeutungen haben.

Gegenstand der Erfindung sind weiter vorzugsweise Verbindungen der Formel (I), in welcher
$R^1$ für Wasserstoff, für gegebenenfalls durch Fluor, Cyano, Methoxy oder Ethoxy substituiertes $C_1$-$C_4$-Alkyl, für Allyl, für $C_3$-$C_6$-Cycloalkyl, für Phenyl, für $C_1$-$C_4$-Alkoxy, $C_3$-$C_4$-Alkenyloxy, für $C_1$-$C_3$-Alkylamino, $C_3$-$C_6$-Cycloalkylamino oder für Di-($C_1$-$C_3$-alkyl)-amino steht,
$R^2$ für Chlor oder Brom steht, und
$R^3$ für die Gruppierung

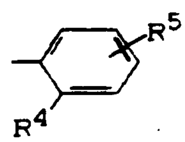

steht, worin
$R^4$ für Fluor, Chlor, Brom, Methyl, Trifluormethyl, Methoxy, Difluormethoxy, Trifluormethoxy, 2-Chlorethoxy, 2-Methoxy-ethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, N-Methoxyaminosulfonyl, Phenyl, Phenoxy oder $C_1$-$C_3$-Alkoxy-carbonyl steht und
$R^5$ für Wasserstoff, Fluor, Chlor oder Brom steht;
weiterhin
$R^3$ für den Rest

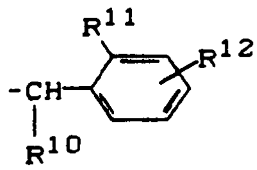

steht, worin

$R^{10}$ für Wasserstoff steht,

$R^{11}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{12}$ für Wasserstoff steht;

weiterhin

$R^3$ für den Rest

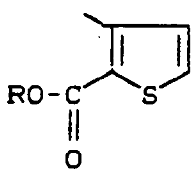

steht,
worin R für $C_1$-$C_4$-Alkyl steht, oder
für den Rest

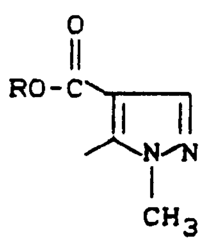

steht,
worin R für $C_1$-$C_4$-Alkyl steht.

Beispiele für die erfindungsgemäßen Verbindungen sind in der nachstehenden Tabelle 1 aufgeführt - vgl. auch die Herstellungsbeispiele.

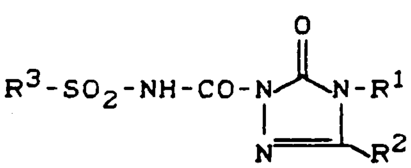 (I)

Tabelle 1: Beispiele für die Verbindungen der Formel (I)

| $R^1$ | $R^2$ | $R^3$ |
|---|---|---|
| 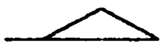 | Br | 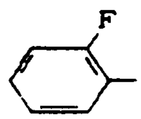 |
| 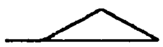 | Cl | 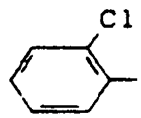 |
| $CH_3$ | F | 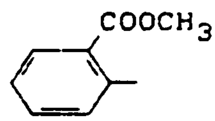 |
| $CH_3$ | Cl | 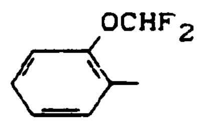 |
| $CH_3$ | Br | 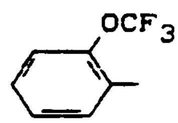 |
| $C_2H_5$ | F | 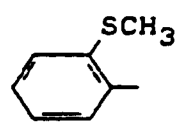 |
| $CH_2-CH=CH_2$ | Cl | 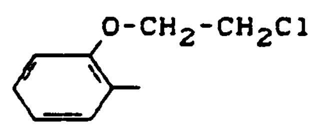 |

Tabelle 1: (Fortsetzung)

| $R^1$ | $R^2$ | $R^3$ |
|---|---|---|
| 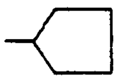 | Cl | 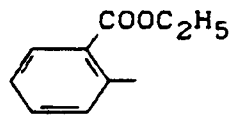 |
| 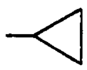 | Br | 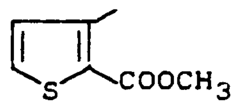 |
| 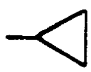 | Cl | 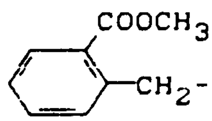 |
| $CH_3$ | Br | 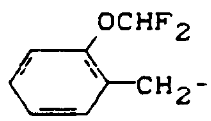 |
| $CH_3$ | Br | 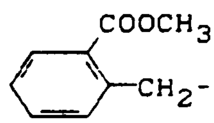 |
| $C_2H_5$ | Cl | 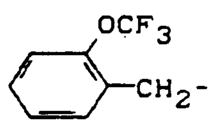 |
| $OCH_3$ | Cl | 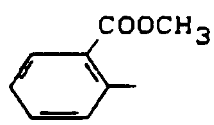 |
| $O-CH_2-CH=CH_2$ | Cl | 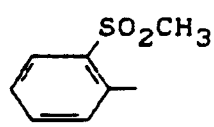 |

Tabelle 1: (Fortsetzung)

| $R^1$ | $R^2$ | $R^3$ |
|---|---|---|
| $CH_3$ | Cl | 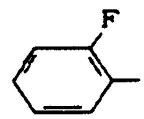 |
| $CH_3$ | Br | 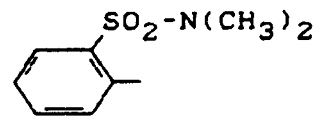 |
| $CH_3$ | Cl | 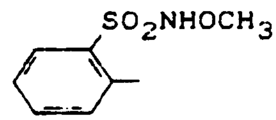 |
| $C_2H_5$ | Cl | 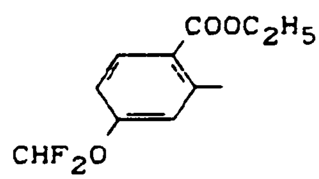 |
| $C_3H_7$ | Cl | 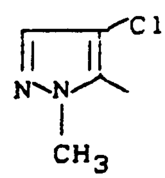 |
| 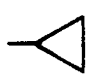 | Cl | 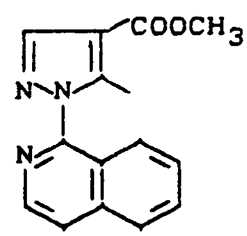 |
| $CH_3$ | Cl | 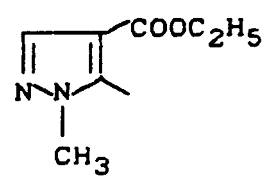 |

Tabelle 1: (Fortsetzung)

| $R^1$ | $R^2$ | $R^3$ |
|---|---|---|
| $N(CH_3)_2$ | Cl | 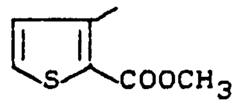 |
| $N(CH_3)_2$ | F | 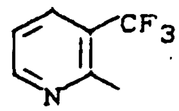 |
| $CH_3$ | Cl | 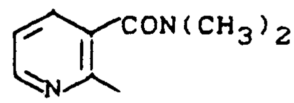 |
| $CH_3$ | Cl | 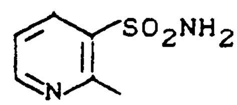 |
| $C_2H_5$ | Cl | 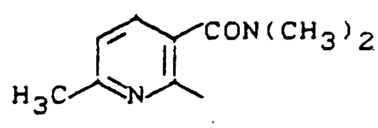 |
| 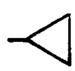 | Br | 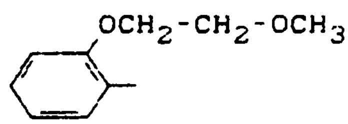 |
| 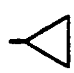 | Cl | 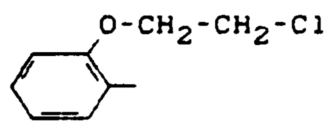 |
| 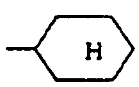 | F | 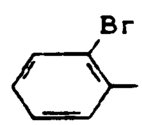 |
| 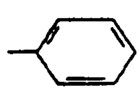 | Cl | 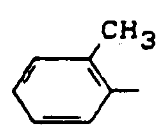 |

Tabelle 1: (Fortsetzung)

| $R^1$ | $R^2$ | $R^3$ |
|---|---|---|
| $CH_2$ 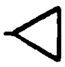 | Br | 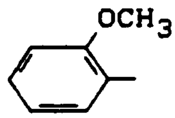 |
| 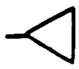 | Cl | 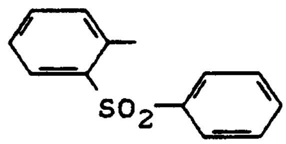 |
| $CH_3$ | Br | 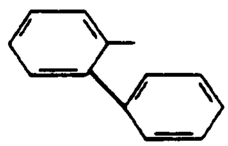 |
| $CH_3$ | Cl | 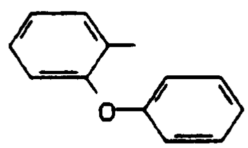 |
| $N(CH_3)_2$ | Cl | 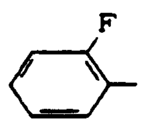 |
| $CH_3$ | Cl | 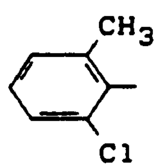 |
| $CH_3$ | Br | 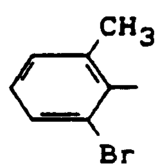 |

Tabelle 1: (Fortsetzung)

| $R^1$ | $R^2$ | $R^3$ |
|---|---|---|
| $CH_3$ | F | 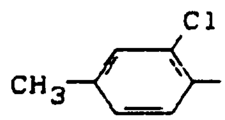 |
| 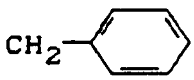 | Cl | 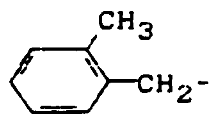 |
| $CH_3$ | Cl | 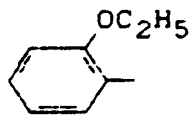 |
| $CH_3$ | Br | 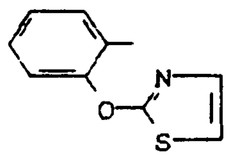 |
| 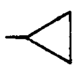 | Cl | 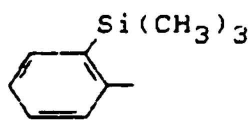 |
| 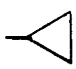 | Br | 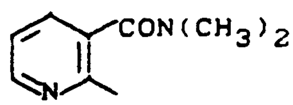 |
| 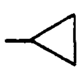 | Cl | 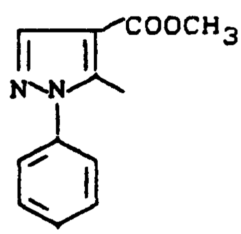 |
| $CH_2$-CH=$CH_2$ | Br | 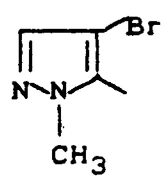 |

Tabelle 1: (Fortsetzung)

| $R^1$ | $R^2$ | $R^3$ |
|---|---|---|
| $CH_2-CHBr-CH_2-Br$ | Br | 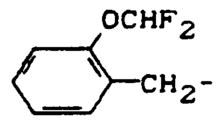 |
| $CH_3$ | Cl | 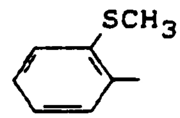 |
| $CH_3$ | Br | 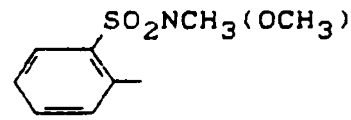 |
| $CH_3$ | Cl | 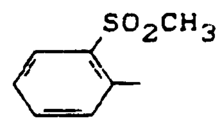 |
| $CH_3$ | F | 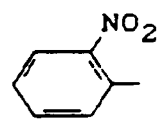 |
| $C_2H_5$ | Cl | 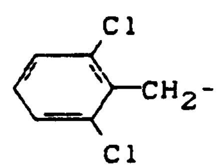 |
| $C_2H_5$ | Br | 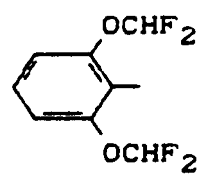 |
| $C_3H_7$-n | Br | 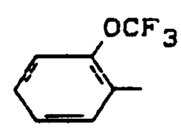 |

Tabelle 1: (Fortsetzung)

| $R^1$ | $R^2$ | $R^3$ |
|---|---|---|
| $CH_3$ | Cl | 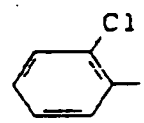 |
| $CH_3$ | Br | 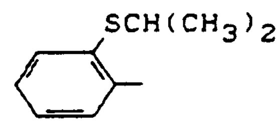 |

Verwendet man beispielsweise 2,6-Difluor-phenylsulfonylisocyanat und 5-Brom-4-ethyl-2,4-dihydro-3H-1,2,4-triazol-3-on als Ausgangsstoffe, so kann der Reaktionsablauf beim erfindungsgemäßen Verfahren (a) durch folgendes Formelschema skizziert werden:

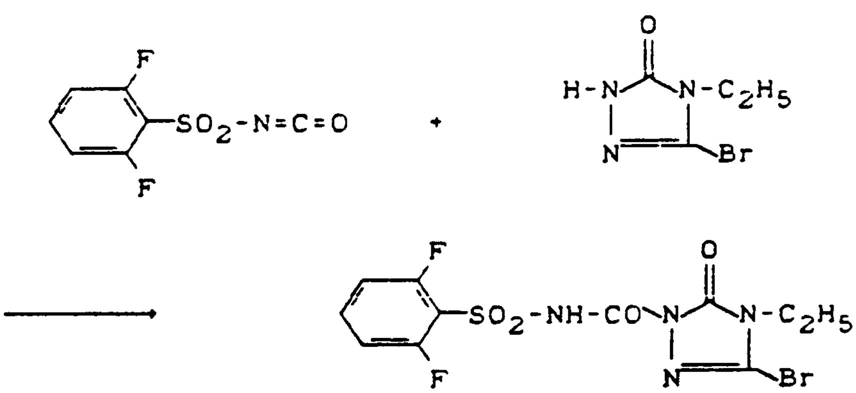

Verwendet man beispielsweise 2-Methoxy-benzolsulfonsäureamid und 5-Chlor-2-chlorcarbonyl-4-methoxy-2,4-dihydro-3H-1,2,4-triazol-3-on als Ausgangsstoffe, so kann der Reaktionsablauf beim erfindungsgemäßen Verfahren (b) durch folgendes Formelschema skizziert werden:

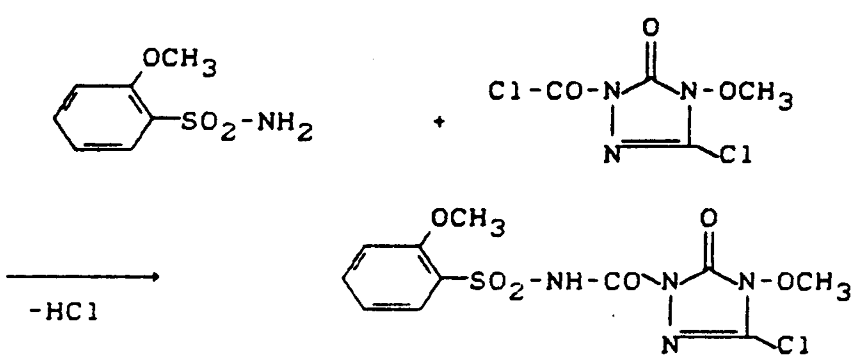


Verwendet man beispielsweise 5-Brom-4-dimethylamino-2,4-dihydro-3H-1,2,4-triazol-3-on und N-Methoxycarbonyl-2-trifluormethyl-benzolsulfonsäureamid als Ausgangsstoffe, so kann der Reaktionsablauf beim erfindinngsgemäßen Verfahren (c) durch das folgende Formelschema skzziert werden:

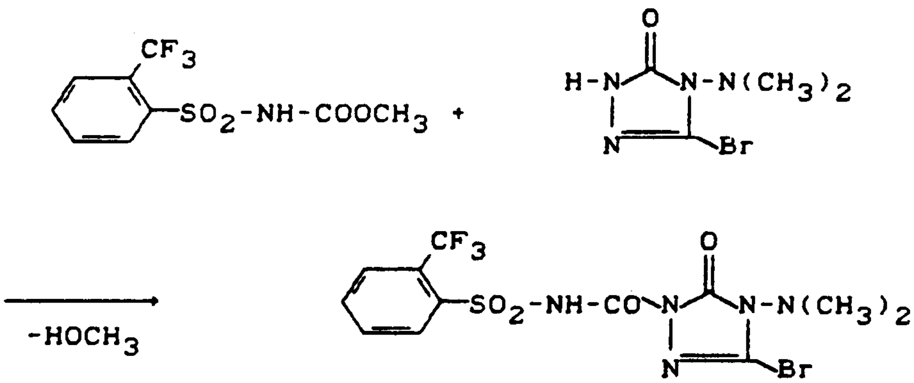


Die bei den erfindungsgemäßen Verfahren (a) und (c) zur Herstellung von Verbindungen der Formel (I) als Ausgangsstoffe zu verwendenden halogenierten Triazolinone sind durch die Formel (II) allgemein definiert.

In Formel (II) haben $R^1$ und $R^2$ vorzugsweise diejenigen Bedeutungen, die bereits oben im Zusammenhang mit der Beschreibung der erfindungsgemäßen Verbindungen der Formel (I) vorzugsweise für $R^1$ und $R^2$ angegeben wurden,

Beispiele für die Ausgangsstoffe der Formel (II) sind in der nachstehenden Tabelle 2 aufgeführt.

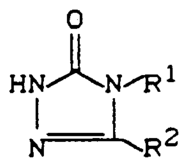

(II)

Tabelle 2: Beispiele für die Ausgangsstoffe der Formel (II)

| $R^1$ | $R^2$ |
|---|---|
| H | Br |
| $CH_3$ | Br |
| $C_2H_5$ | Br |
| $C_3H_7$ | Br |
| $CH(CH_3)_2$ | Cl |
| $C_4H_9$ | Cl |
| $CH_3$ | Cl |
| $C_2H_5$ | Cl |
| 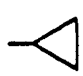 | Br |
| $CH_2-CH=CH_2$ | Br |

Tabelle 2: (Fortsetzung)

| $R^1$ | $R^2$ |
|---|---|
| $CH(CH_3)_2$ | F |
| $CH_3$ | F |
| $C_3H_7$ | Cl |
| $C_2H_5$ | F |
| 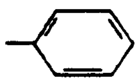 | Br |
| $N(CH_3)_2$ | Br |
| $N(CH_3)_2$ | Cl |
| $NH-CH_3$ | Br |
| $OCH_3$ | Br |
| $OCH_3$ | Cl |
| $C_2H_5$ | F |
| $C_3H_7$ | F |
| 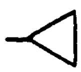 | Cl |
| 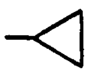 | F |
| $OC_2H_5$ | Br |
| $OC_2H_5$ | Cl |
| $CH_2$ 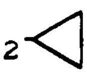 | Br |
| $CH_2$ 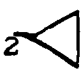 | Cl |
| $C(CH_3)_3$ | Br |
| $CH_2-CH(CH_3)_2$ | Br |
| $CH_2-CH(CH_3)_2$ | Cl |
| $CH_2$ 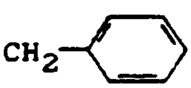 | Br |
| $O-C_3H_7-n$ | Br |

Tabelle 2: (Fortsetzung)

| $R^1$ | $R^2$ |
|---|---|
| $CH_2$ 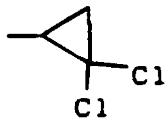 | Br |
| $C_2H_5$ | I |
| $C_4H_9$ | Br |
| $CH(CH_3)-C_2H_5$ | Br |
| $CH(CH_3)-C_2H_5$ | Cl |
| $CH(CH_3)_2$ | Cl |
| $CH_2-CHBr-CH_2Br$ | Br |
| $CH_2-CHBr-CH_2Br$ | Cl |
| 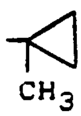 | Br |
| 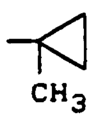 | Cl |
| 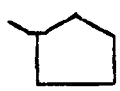 | F |
| 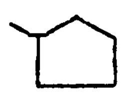 | Cl |
| 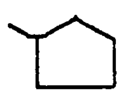 | Br |

Tabelle 2: (Fortsetzung)

| $R^1$ | $R^2$ |
|---|---|
| 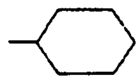 | Br |
| 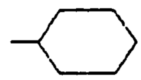 | Cl |
| 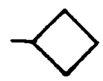 | Br |
| 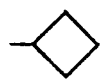 | Cl |
| $OC_3H_7$ | Cl |

Die Ausgangsstoffe der Formel (II) sind bekannt und/oder können nach an sich bekannten Verfahren hergestellt werden (vgl. Chem. Ber. 102 (1969), 755-766 und die Herstellungsbeispiele).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In Formel (III) hat $R^3$ vorzugsweise diejenige Bedeutung, die bereits oben im Zusammenhang mit der Beschreibung der erfindungsgemäßen Verbindungen der Formel (I) vorzugsweise für $R^3$ angegeben wurde.

Als Beispiele für die Ausgangsstoffe der Formel (III) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-phenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonylisocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 80 °C.

Das erfindungsgemäße Verfahren (a) wird im allgemeinen bei Normaldruck durchgeführt.

Zur Durchführung des erfindinngsgemäßen Verfahrens (a) setzt man je Mol Triazolinon der Formel (II) im allgemeinen zwischen 1 und 3 Mol, vorzugsweise zwischen 1 und 2 Mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt, eingeengt und das im Rückstand verbleibende Rohprodukt mit einem geeigneten Lösungsmittel, wie z. B. Diethylether, zur Kristallisation gebracht. Das kristallin angefallene Produkt der Formel (I) wird durch Absaugen isoliert.

Die beim erfindungsgemäßen Verfahren (b) zur Herstellung von Verbindungen der Formel (I) als Ausgangsstoffe zu verwendenden Triazolinon-Derivate sind durch die Formel (IV) allgemein definiert.

In Formel (IV) haben $R^1$ und $R^2$ vorzugsweise diejenigen Bedeutungen, die bereits oben im Zusammenhang mit der Beschreibung der erfindungsgemäßen Verbindungen der Formel (I) vorzugsweise bevorzugt für $R^1$ und $R^2$ angegeben wurden und

Z steht vorzugsweise für Methoxy oder Phenoxy.

Mögliche Beispiele für die Ausgangsstoffe der Formel (IV) sind die aus den in Tabelle 2 aufgeführten Verbindungen der Formel (II) und Phosgen, Chlorameisensäuremethylester, Chlorameisensäure-benzylester, Chlorameisensäurephenylester oder Diphenylcarbonat herzustellenden Verbindungen der Formel (IV).

Die Ausgangsstoffe der Formel (IV) sind noch nicht bekannt.

Man erhält die neuen halogenierten Triazolinon-Derivate der Formel (IV), wenn man Triazolinone der allgemeinen Formel (II)

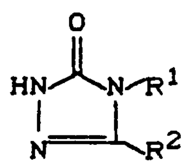

(II)

in welcher
$R^1$ und $R^2$ die oben angegebenen Bedeutungen haben,
mit Kohlensäurederivaten der allgemeinen Formel (XI)

$Z\text{-}CO\text{-}Z^1$ (XI)

in welcher

Z die oben angegebene Bedeutung hat und

$Z^1$ für eine Abgangsgruppe wie Chlor, Methoxy, Benzyloxy oder Phenoxy steht,

gegebenenfalls in Gegenwart eines Verdünnungsmittels, wie z.B. Tetrahydrofuran, und gegebenenfalls in Gegenwart eines Säureakzeptors, wie z.B. Natriumhydrid oder Kalium-tert-butylat, bei Temperaturen zwischen -20 °C und +100 °C umsetzt (vgl. auch die Herstellungsbeispiele).

Die beim erfindungsgemäßen Verfahren (b) zur Herstellung von Verbindungen der Formel (I) weiter als Ausgangsstoffe zu verwendenden Sulfonsäureamide sind durch die Formel (V) allgemein definiert,

In Formel (V) hat $R^3$ vorzugsweise diejenige Bedeutung, die bereits oben im Zusammenhang mit der Beschreibung der erfindungsgemäßen Verbindungen der Formel (I) vorzugsweise für $R^3$ angegeben wurde.

Als Beispiele für die Ausgangsstoffe der Formel (V) seien genannt:

2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-benzolsulfonsäureamid, 2-Fluor-, 2-Chlor-, 2-Difluormethoxy-, 2-Trifluormethoxy-, 2-Methoxycarbonyl- und 2-Ethoxycarbonyl-phenylmethansulfonsäureamid, 2-Methoxycarbonyl-3-thiophensulfonsäureamid, 4-Methoxycarbonyl-und 4-Ethoxycarbonyl-1-methyl-pyrazol-5-sulfonsäureamid.

Die Sulfonsäureamide der Formel (V) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Betracht, wie sie beispielsweise oben für das erfindungsgemäße Verfahren (a) angegeben sind.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden halogenierten Triazolinone der Formel (II) sind bereits als Ausgangsstoffe für das erfindungsgemäße Verfahren (a) beschrieben worden.

Die beim erfindungsgemäßen Verfahren (c) zur Herstellung von Verbindungen der Formel (I) weiter als Ausgangsstoffe zu verwendenden Sulfonsäureamid-Derivate sind durch die Formel (VI) allgemein definiert.

In Formel (VI) haben $R^3$ und Z vorzugsweise diejenigen Bedeutungen, die bereits oben im Zusammenhang mit der Beschreibung der erfindungsgemäßen Verbindungen der Formel (I) bzw. (IV) vorzugsweise für $R^3$ und Z angegeben wurden.

Das erfindungsgemäße Verfahren (c) wird vorzugsweise unter Verwendung von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (c) wird gegebenenfalls in Gegenwart eines Säureakzeptors durchgeführt. Es kommen hierbei die gleichen Säurebindemittel in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Zur Überführung in Salze werden die Verbindungen der Formel (I) mit geeigneten Salzbildnern, wie z.B. Natrium- oder Kalium-hydroxid, -methylat oder -ethylat, Ammoniak, Isopropylamin, Dibutylamin oder Triethylamin, in geeigneten Verdünnungsmitteln, wie z.B. Wasser, Methanol oder Ethanol, verrührt. Die Salze können - dann gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf - als auch im Nachauflauf-Verfahren. Sie sind deutlich wirksamer als z.B. Isocarbamid.

In gewissem Umfang zeigen die erfindungsgemäßen Verbindungen auch fungizide Wirkung, z.B. gegen echten Mehltau an Reben und gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 4-Amino-6-t-butyl- 3-ethylthio-1,2,4-triazin-5(4H)-on (ETHIOZIN); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ): 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA): (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino)-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

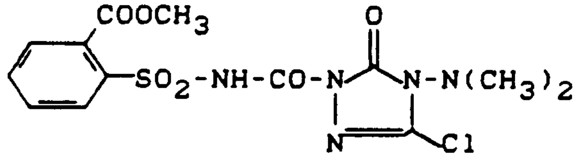

(Verfahren (a))

2,8 g (17,2 mMol) 5-Chlor-4-dimethylamino-2,4-dihydro-3H-1,2,4-triazol-3-on werden in 60 ml Acetonitril gelöst und unter Rühren werden 6,6 g (27,4 mMol) 2-Methoxycarbonyl-phenylsulfonylisocyanat, gelöst in 20 ml Acetonitril, zu dieser Lösung gegeben. Das Reaktionsgemisch wird 6 Stunden bei 20 °C gerührt und dann eingeengt. Der verbleibende Rückstand wird mit Diethylether verrührt, und das kristallin angefallene Produkt wird durch Absaugen isoliert.

Man erhält 6,9 g (99 % der Theorie) 5-Chlor-4-dimethylamino-2-(2-methoxycarbonyl-phenylsulfonylaminocarbonyl)-2,4-dihydro-3H-1,2,4-triazol-3-on vom Schmelzpunkt 146 °C.

Beispiel 2

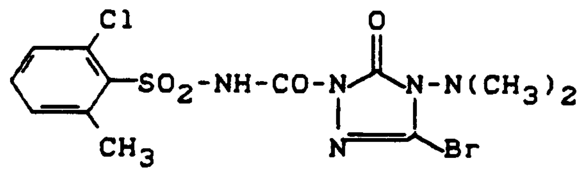

(Verfahren (b))

1,9 g (12,5 mMol) 1,8-Diazabicyclo-[5,4,0]-undec-7-en (DBU) und 2,6 g (12,6 mMol) 2-Chlor-6-methylbenzolsulfonamid werden zu einer Lösung von 4,0 g (12,2 mMol) 5-Brom-4-dimethylamino-2-phenoxycarbonyl-2,4-dihydro-3H-1,2,4-triazol-3-on in 60 ml Acetonitril gegeben. Das Reaktionsgemisch wird 3 Stunden bei 20 °C gerührt, dann auf etwa das doppelte Volumen Eiswasser gegossen und durch Zugabe von 2N-Salzsäure der pH-Wert auf ca. 2 eingestellt. Anschließend wird mit Methylenchlorid extrahiert, die organische Phase mit Wasser gewaschen, mit Natriumsulfat getrocknet und filtriert. Das Filtrat wird eingeengt, der Rückstand mit Diethylether verrührt und das kristallin angefallene Produkt durch Absaugen isoliert.

Man erhält 1,0 g (19 % der Theorie) 5-Brom-4-dimethylamino-2-(2-chlor-6-methyl-phenylsulfonyl-aminocarbonyl)-2,4-dihydro-3H-1,2,4-triazol-3-on vom Schmelzpunkt 163 °C.

Analog zu den Beispielen 1 und 2 sowie entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

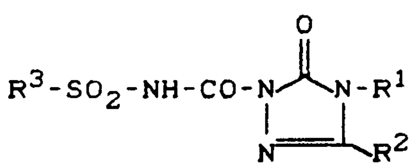

(I)

Tabelle 3:

Herstellungsbeispiele für die Verbindungen der Formel (I)

| Bsp. Nr. | $R^1$ | $R^2$ | $R^3$ | Schmelzpunkt (°C) |
|---|---|---|---|---|
| 3 | $CH_3$ | Cl | 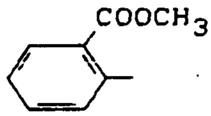 | 170 |
| 4 | $CH_3$ | Br | 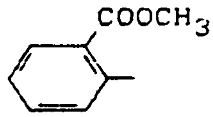 | 173 |
| 5 | $N(CH_3)_2$ | Br | 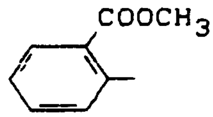 | 139 |
| 6 | 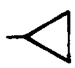 | Br | 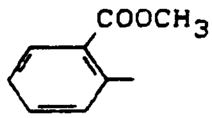 | 155 |
| 7 | 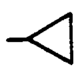 | Cl | 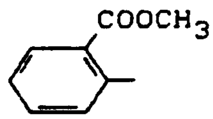 | 148 |
| 8 | $C_3H_7$ | Br | 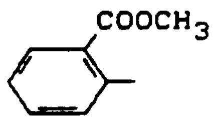 | 120 |
| 9 | $C_3H_7$ | Cl | 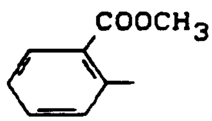 | 119 |

Tabelle 3 (Fortsetzung)

| Bsp. Nr. | $R^1$ | $R^2$ | $R^3$ | Schmelzpunkt (°C) |
|---|---|---|---|---|
| 10 | $CH_3$ | Cl | 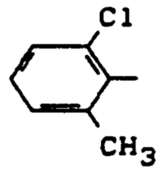 | 141 |
| 11 | $CH_3$ | Br | 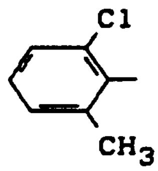 | 159 |
| 12 | 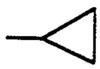 | Br | 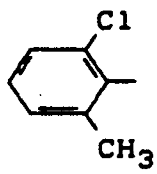 | 168 |
| 13 | 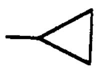 | Cl | 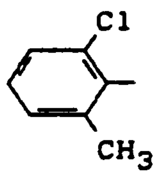 | 162 |
| 14 | $C_2H_5$ | Br | 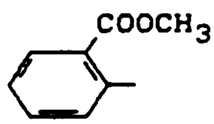 | 147 |
| 15 | $C_2H_5$ | Cl | 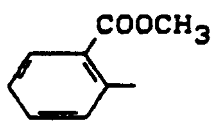 | 161 |
| 16 | $C_2H_5$ | Br | 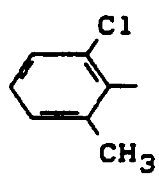 | 161 |

Tabelle 3 (Fortsetzung)

| Bsp. Nr. | $R^1$ | $R^2$ | $R^3$ | Schmelzpunkt (°C) |
|---|---|---|---|---|
| 17 | $C_2H_5$ | Cl | 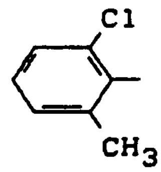 | 156 |
| 18 | 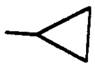 | Cl | 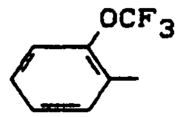 | 142 |
| 19 | 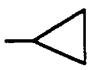 | Br | 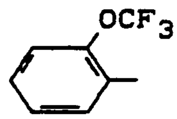 | 134 |
| 20 | $CH_3$ | Cl | 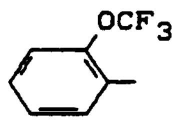 | 144 |
| 21 | $CH_3$ | Br | 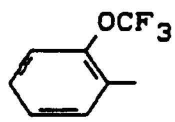 | 169 |
| 22 | $C_2H_5$ | Cl | 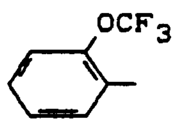 | 122 |
| 23 | $C_2H_5$ | Br | 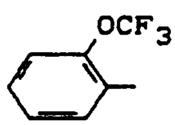 | 133 |
| 24 | 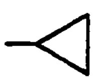 | Cl | 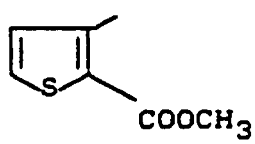 | 174 |

Ausgangsstoffe der Formel (II):

Beispiel (II-1)

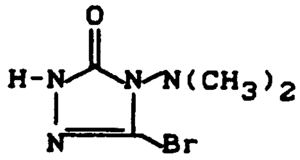

Stufe 1:

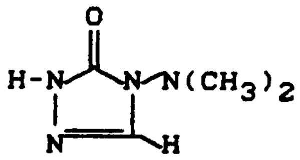

856 g (4,0 Mol) Diphenylcarbonat werden in 588 g Ethylenchlorid gelöst. Unter Wasserkühlung werden 245 g (4,0 Mol) Dimethylhydrazin (98%ig) zugetropft, dann wird langsam erwärmt und nach 4 Stunden bei 60 ° C nachgerührt.

Nach Abkühlen auf 20 ° C werden 200 g (4,0 Mol) Hydrazinhydrat zugetropft und 12 Stunden nachgerührt. Es wird auf 70-80 ° C erwärmt und nach ca. 1 Stunde weiter gerührt. Die erkaltete Lösung wird im Vakuum destilliert, wobei Ethylenchlorid und Wasser entfernt werden (Sumpftemperatur zuletzt 100 ° C). Zu 424 g (4,0 Mol) Orthoameisensäure-trimethylester wird im Verlauf von 90 Minuten bei Rückflußtemperatur (ca. 102 ° C) obige phenolische Dimethylcarbodihydrazid-Lösung getropft. Nach destillativem Entfernen des gebildeten Methanols wird im Vakuum Phenol abdestilliert, anschließend werden bei einer Kopftemperatur von 85-105 ° C 282 g Produktgemisch erhalten. Dieses Gemisch wird mit 600 ml Aceton verkocht und nach Filtration in der Siedehitze wird das Filtrat abgekühlt. Das dabei kristallin angefallene Produkt wird durch Absaugen isoliert.

Man erhält 71 g (14% der Theorie) 4-Dimethylamino-2,4-dihydro-3H-1,2,4-triazol-3-on vom Schmelzpunkt 127 ° C.

Stufe 2:

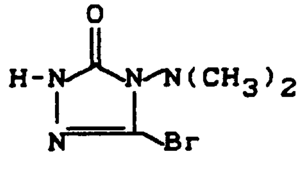

Eine Mischung aus 15,0 g (0,117 Mol) 4-Dimethylamino-2,4-dihydro-3H-1,2,4-triazol-3-on, 4,7 g (0,117 Mol) Natriumhydroxid und 150 ml Waser wird unter Eiskühlung und unter Rühren im Verlauf von 2 Stunden mit 18,8 g (0,117 Mol) Brom versetzt. Das kristallin angefallene Produkt wird anschließend durch Absaugen isoliert.

Man erhält 19,6 g (81 % der Theorie) 5-Brom-4-dimethylamino-2,4-dihydro-3H-1,2,4-triazol-3-on vom Schmelzpunkt 163 ° C.

Beispiel (II-2)

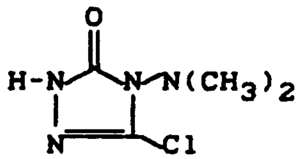

Eine Mischung aus 6,0 g (0,029 Mol) 5-Brom-4-dimethylamino-2,4-dihydro-3H-1,2,4-triazol-3-on und 200 ml konzentrierter Salzsäure wird 3 Stunden unter Rückfluß erhitzt. Dann wird eingeengt, der Rückstand in wenig Wasser aufgenommen, mit Natriumhydrogencarbonat neutralisiert und das kristallin angefallene Produkt durch Absaugen isoliert. Das Filtrat wird mit Essigsäureethylester extrahiert, das vorher isolierte kristalline Produkt zur organischen Phase gegeben, letztere mit Natriumsulfat getrocknet und filtriert. Das Filtrat wird eingeengt, der Rückstand mit Diethylether verrieben und das hierbei kristallin angefallene Produkt durch Absaugen isoliert.

Man erhält 3,1 g (66 % der Theorie) 5-Chlor-4-dimethylamino-2,4-dihydro-3H-1,2,4-triazol-3-on vom Schmelzpunkt 158 ° C.

Analog zu den Beispielen (II-1) unds (II-2) können beispielsweise auch die in der nachstehenden Tabelle 4 aufgeführten Verbindungen der Formel (II) hergestellt werden.

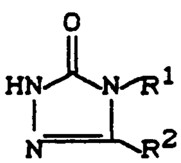

(II)

**Tabelle 4:** Herstellungsbeispiele für die Verbindungen der Formel (II)

| Bsp.-Nr. | $R^1$ | $R^2$ | Schmelzpunkt (°C) |
|---|---|---|---|
| II-3 | 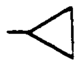 | Br | 137 |
| II-4 | $C_3H_7$ | Br | 98 |
| II-5 | 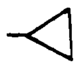 | Cl | 121 |
| II-6 | $C_3H_7$ | Cl | 91 |
| II-7 | $C_2H_5$ | Br | 142 |
| II-8 | $C_2H_5$ | Cl | 112 |
| II-9 | $CH_2$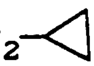 | Br | |
| II-10 | $CH_2$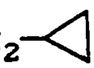 | Cl | |

Ausgangsstoffe der Formel (IV):

Beispiel (IV-1)

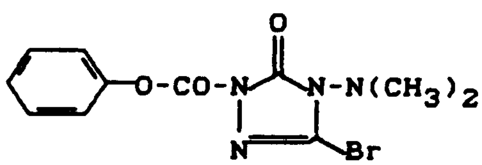

7,0 g (33,8 mMol) 5-Brom-4-dimethylamino-2,4-dihydro-3H-1,2,4-triazol-3-on werden in 100 ml Wasser und 100 ml Methylenchlorid aufgenommen und mit 0,2 g Tetrabutylammoniumbromid und 1,5 g (37,5 mMol) Natriumhydroxid versetzt. Unter starkem Rühren werden dann bei 20 °C 5,9 g (37,7 mMol) Chlorameisensäurephenylester zugetropft und das Reaktionsgemisch wird noch 12 Stunden bei 20 °C gerührt. Die organische Phase wird abgetrennt, mit Wasser gewaschen, mit Natriumsulfat getrocknet und filtriert. Das Filtrat wird eingeengt, der Rückstand mit Diethylether verrührt und das hierbei kristallin angefallene Produkt durch Absaugen isoliert.

Man erhält 8,7 g (79 % der Theorie) 5-Brom-4-dimethylamino-2-phenoxycarbonyl-2,4-dihydro-3H-1,2,4-triazol-3-on vom Schmelzpunkt 136 °C.

Anwendungsbeispiele:

Bei den folgenden Anwendungsbeispielen wird das bekannte Herbizid Isocarbamid nachstehender Formel (A) als Vergleichssubstanz herangezogen:

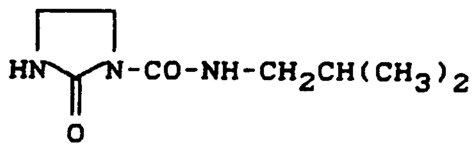

(A)

(bekannt aus BE 737 449; DE 1 795 117).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß Herstellungsbeispiel 3, 6 und 7.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß Herstellungsbeispiel 3, 6 und 7.

**Patentansprüche**
**Patentansprüche für folgende Vertragsstaaten : BE, CH, DE, DK, FR, GB, IT, LI, NL**

1. Halogenierte Sulfonylaminocarbonyltriazolinone der allgemeinen Formel (I),

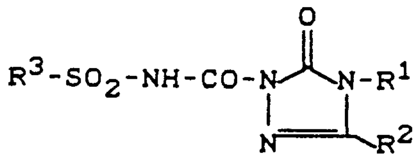

(I)

in welcher

$R^1$ für Wasserstoff, Hydroxy, Amino, für gegebenenfalls durch Fluor, Chlor, Brom, Cyano, $C_1$-$C_4$-Alkoxy, $C_1$-$C_4$-Alkylcarbonyl oder $C_1$-$C_4$-Alkoxy-carbonyl substituiertes $C_1$-$C_6$-Alkyl, für jeweils gegebenenfalls durch Fluor, Chlor und/oder Brom substituiertes $C_3$-$C_6$-Alkenyl oder $C_3$-$C_6$-Alkinyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio, $C_1$-$C_4$-Alkylsulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für $C_3$-$C_4$-Alkenyloxy, für gegebenenfalls durch Fluor, Cyano, Phenyl, $C_1$-$C_4$-Alkoxy oder $C_1$-$C_4$-Alkoxy-carbonyl substituiertes $C_1$-$C_4$-Alkylamino, $C_3$-$C_6$-Cycloalkylamino oder für Di-($C_1$-$C_4$-alkyl)-amino steht,

$R^2$ für Fluor, Chlor, Brom oder Jod steht und

$R^3$ für die Gruppierung

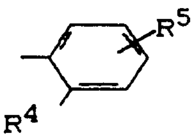

steht, worin $R^4$ und $R^5$ gleich oder verschieden sind und für Wasserstoff, Fluor, Chlor, Brom, Iod, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkylcarbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^6$ stehen, wobei

p für die Zahlen 1 oder 2 steht und

$R^6$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino, Phenyl oder für den Rest -NHOR$^7$ steht, wobei

$R^7$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^4$ und/oder $R^5$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest -CO-R$^8$ stehen, wobei

$R^8$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$ Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^4$ und/oder $R^5$ weiterhin für Trimethylsilyl, Thiazolinyl, $C_1$-$C_4$-Alkylsulfonyloxy, Di-($C_1$-$C_4$-alkyl)-aminosulfonylamino oder für den Rest -CH = N-R$^9$ stehen, wobei

$R^9$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiterhin

$R^3$ für den Rest

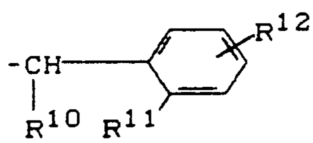

steht, worin

$R^{10}$ für Wasserstoff oder $C_1$-$C_4$-Alkyl steht,

$R^{11}$ und $R^{12}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy, $C_1$-$C_4$-Alkoxy-carbonyl, Dimethylaminocarbonyl, $C_1$-$C_4$-Alkylsulfonyl oder Di-($C_1$-$C_4$-alkyl)-aminosulfonyl stehen;

weiterhin

$R^3$ für den Rest

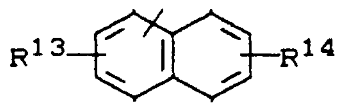

steht,
worin

$R^{13}$ und $R^{14}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiterhin
$R^3$ für den Rest

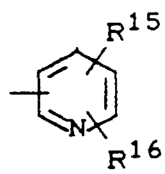

steht, worin
$R^{15}$ und $R^{16}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/ oder Chlor substituiert sind), für Aminosulfonyl, Mono-($C_1$-$C_4$-alkyl)-aminosulfonyl, Di-($C_1$-$C_4$- alkyl)-aminosulfonyl oder $C_1$-$C_4$-Alkoxy-carbonyl oder Dimethylaminocarbonyl stehen;

weiterhin
$R^3$ für den Rest

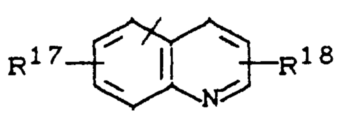

steht, worin
$R^{17}$ und $R^{18}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

weiterhin
$R^3$ für den Rest

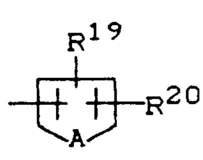

steht, worin

$R^{19}$ und $R^{20}$ gleich oder verschieden sind und für Wasserstoff, Fluor, Chlor, Brom, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/ oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl, $C_1$-$C_4$-Alkoxy-carbonyl oder Dimethylaminocarbonyl stehen, und

A für Sauerstoff, Schwefel oder die Gruppierung N-$Z^1$ steht, wobei $Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl Benzyl, Phenyl, (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiterhin

$R^3$ für den Rest

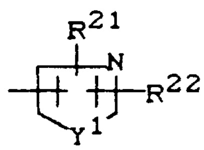

steht, worin

$R^{21}$ und $R^{22}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{23}$ steht, wobei

$R^{23}$ für Wasserstoff oder $C_1$-$C_4$-Alkyl steht;

weiterhin

$R^3$ für den Rest

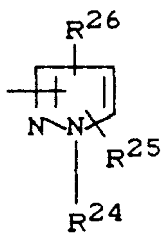

steht, worin

$R^{24}$ für Wasserstoff, $C_1$-$C_4$-Alkyl, Benzyl, Pyridyl, Chinolinyl oder Phenyl steht,

$R^{25}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist) Dioxolanyl oder $C_1$-$C_4$-Alkoxy-carbonyl steht und

$R^{26}$ für Wasserstoff, Halogen oder $C_1$-$C_4$-Alkyl steht;

weiterhin

$R^3$ für eine der nachstehend aufgeführten Gruppierungen steht:

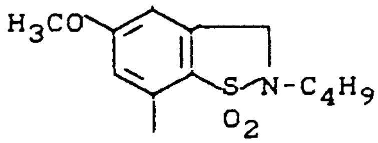 ; 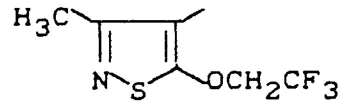

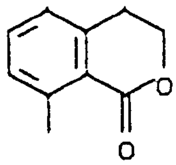

sowie Salze von Verbindungen der Formel (I).

**2.** Halogenierte Sulfonylaminocarbonyltriazolinone der allgemeinen Formel (I) gemaß Anspruch 1, in welcher

$R^1$ für Wasserstoff, für gegebenenfalls durch Fluor, Cyano, Methoxy oder Ethoxy substituiertes $C_1$-$C_4$-Alkyl, für Allyl, für $C_3$-$C_6$-Cycloalkyl, für Phenyl, für $C_1$-$C_4$-Alkoxy, $C_3$-$C_4$-Alkenyloxy, für $C_1$-$C_3$-Alkylamino, $C_3$-$C_6$-Cycloalkylamino oder für Di-($C_1$-$C_3$-alkyl)-amino steht,

$R^2$ für Chlor oder Brom steht, und

$R^3$ für die Gruppierung

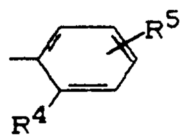

steht, worin

$R^4$ für Fluor, Chlor, Brom, Methyl, Trifluormethyl, Methoxy, Difluormethoxy, Trifluormethoxy, 2-Chlor-ethoxy, 2-Methoxy-ethoxy, $C_1$-$C_3$-Alkylthio, $C_1$-$C_3$-Alkylsulfinyl, $C_1$-$C_3$-Alkyl-sulfonyl, Dimethylaminosulfonyl, Diethylaminosulfonyl, N-Methoxy-N-methylaminosulfonyl, N-Methoxyaminosulfonyl, Phenyl, Phenoxy oder $C_1$-$C_3$-Alkoxy-carbonyl steht und

$R^5$ für Wasserstoff, Fluor, Chlor oder Brom steht;

weiterhin

$R^3$ für den Rest

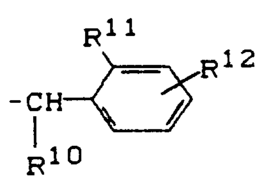

steht, worin

$R^{10}$ für Wasserstoff steht,

$R^{11}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{12}$ für Wasserstoff steht;

weiterhin

$R^3$ für den Rest

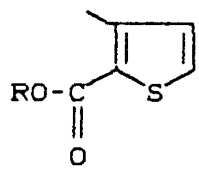

steht,
worin R für $C_1$-$C_4$-Alkyl steht, oder
für den Rest

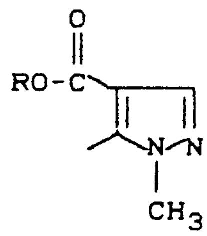

steht,
worin R für $C_1$-$C_4$-Alkyl steht,
sowie Salze von Verbindungen der Formel (I).

**3.** Verfahren zur Herstellung von halogenierten Sulfonylaminocarbonyltriazolinonen der allgemeinen Formel (I) gemäß Anspruch 1 sowie von Salzen von Verbindungen der Formel (I),
dadurch gekennzeichnet, daß man
a) halogenierte Triazolinone der allgemeinen Formel (II)

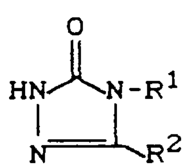

(II)

in welcher
$R^1$ und $R^2$ die in Anspruch 1 angegebenen Bedeutungen haben,
mit Sulfonylisocyanaten der allgemeinen Formel (III),

$R^3$-$SO_2$-N = C = O (III)

in welcher
$R^3$ die in Anspruch 1 angegebene Bedeutung hat,
gegebenenfalls in Gegenwart eines Verdünnungsmittels umsetzt, oder daß man
b) halogenierte Triazolinon-Derivate der allgemeinen Formel (IV)

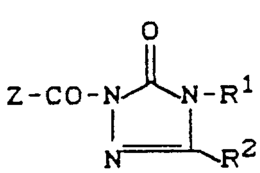

(IV)

in welcher
$R^1$ und $R^2$ die in Anspruch 1 angegebenen Bedeutungen haben und
Z für Chlor, $C_1$-$C_4$-Alkoxy, Benzyloxy oder Phenoxy steht,
mit Sulfonsäureamiden der allgemeinen Formel (V),

$R^3$-$SO_2$-$NH_2$ (V)

in welcher
$R^3$ die in Anspruch 1 angegebene Bedeutung hat,
gegebenenfalls in Gegenwart eines Säureakzeptors und gegebenenfalls in Gegenwart eines Verdünnungsmittels umsetzt, oder daß man
c) halogenierte Triazolinone der allgemeinen Formel (II),

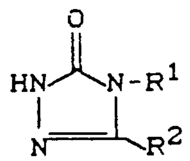

(II)

in welcher
$R^1$ und $R^2$ die in Anspruch 1 angegebenen Bedeutungen haben,
mit Sulfonsäureamid-Derivaten der allgemeinen Formel (VI),

$R^3$-$SO_2$-NH-CO-Z (VI)

in welcher
$R^3$ die in Anspruch 1 angegebene Bedeutung hat und
Z für Chlor, $C_1$-$C_4$-Alkoxy, Benzyloxy oder Phenoxy steht,
gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

**4.** Herbizide Mittel, gekennzeichnet durch einen Gehalt an mindestens einem halogenierten Sulfonylaminocarbonyltriazolinon der Formel (I) gemäß Anspruch 1.

**5.** Verwendung von halogenierten Sulfonylaminocarbonyltriazlinonen der allgemeinen Formel (I) gemäß Anspruch 1 zur Bekämpfung von unerwünschtem Pflanzenwachstum.

**6.** Verfahren zur Bekämpfung von Unkräutern, dadurch gekennzeichnet, daß man halogenierte Sulfonylaminocarbonyltriazolinone der allgemeinen Formel (I) gemäß Anspruch 1 auf die Unkräuter oder ihren Lebensraum einwirken läßt.

**7.** Verfahren zur Herstellung von herbiziden Mitteln, dadurch gekennzeichnet, daß man halogenierte Sulfonylaminocarbonyltriazolinone der allgemeinen Formel (I) gemäß Anspruch 1 mit Streckmitteln und/oder oberflächenaktiven Mitteln vermischt.

**8.** Halogenierte Triazolinone der allgemeinen Formel (IV),

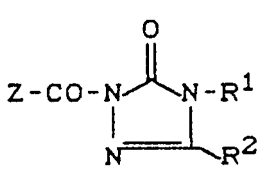

(IV)

in welcher

R[1] und R[2] die in Anspruch 1 angegebenen Bedeutungen haben und

Z für Chlor, $C_1$-$C_4$-Alkoxy, Benzyloxy oder Phenoxy steht.

**Patentansprüche für folgenden Vertragsstaat : ES**

1. Verfahren zur Herstellung von halogenierten Sulfonylaminocarbonyltriazolinonen der allgemeinen Formel (I),

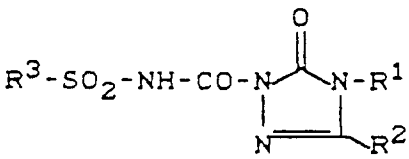

(I)

in welcher

$R^1$ für Wasserstoff, Hydroxy, Amino, für gegebenenfalls durch Fluor, Chlor, Brom, Cyano, $C_1$-$C_4$-Alkoxy, $C_1$-$C_4$-Alkylcarbonyl oder $C_1$-$C_4$-Alkoxy-carbonyl substituiertes $C_1$-$C_6$-Alkyl, für jeweils gegebenenfalls durch Fluor, Chlor und/oder Brom substituiertes $C_3$-$C_6$-Alkenyl oder $C_3$-$C_6$-Alkinyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio, $C_1$-$C_4$-Alkylsulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für $C_3$-$C_4$-Alkenyloxy, für gegebenenfalls durch Fluor, Cyano, Phenyl, $C_1$-$C_4$-Alkoxy oder $C_1$-$C_4$-Alkoxy-carbonyl substituiertes $C_1$-$C_4$-Alkylamino, $C_3$-$C_6$-Cycloalkylamino oder für Di-($C_1$-$C_4$-alkyl)-amino steht,

$R^2$ für Fluor, Chlor, Brom oder Jod steht und

$R^3$ für die Gruppierung

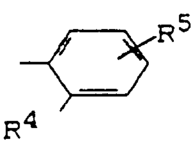

steht, worin $R^4$ und $R^5$ gleich oder verschieden sind und für Wasserstoff, Fluor, Chlor, Brom, Iod, Nitro, $C_1$-$C_6$-Alkyl (welches gegebenenfalls durch Fluor, Chlor, Brom, Cyano, Carboxy, $C_1$–$C_4$-Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$– $C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substitu-

iert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest -$S(O)_p$-$R^6$ stehen, wobei

p für die Zahlen 1 oder 2 steht und

$R^6$ für $C_1$-$C_4$-Alkyl (welches gegebenenfalls durch Fluor, Chlor, Brom, Cyano oder $C_1$- $C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino, Phenyl oder für den Rest -NHOR$^7$ steht, wobei

$R^7$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylcarbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$- $C_4$-Alkoxycarbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^4$ und/oder $R^5$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonyl-amino, Di-($C_1$-$C_4$-alkyl)-amino-carbonylamino, oder für den Rest -CO-$R^8$ stehen, wobei

$R^8$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$ Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^4$ und/oder $R^5$ weiterhin für Trimethylsilyl, Thiazolinyl, $C_1$-$C_4$-Alkylsulfonyloxy, Di-($C_1$-$C_4$-alkyl)-aminosulfonylamino oder für den Rest -CH=N-$R^9$ stehen, wobei

$R^9$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$- $C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$- $C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiterhin

$R^3$ für den Rest

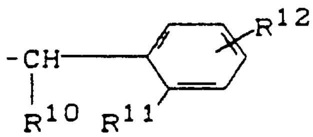

steht, worin

$R^{10}$ für Wasserstoff oder $C_1$-$C_4$-Alkyl steht,

$R^{11}$ und $R^{12}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy, $C_1$-$C_4$-Alkoxy-carbonyl, Dimethylaminocarbonyl, $C_1$-$C_4$-Alkylsulfonyl oder Di-($C_1$-$C_4$-alkyl)-aminosulfonyl stehen;

weiterhin

$R^3$ für den Rest

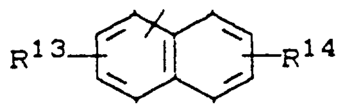

steht,

worin

$R^{13}$ und $R^{14}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiterhin

$R^3$ für den Rest

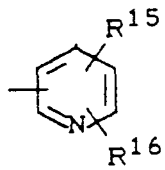

steht, worin

$R^{15}$ und $R^{16}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/ oder Chlor substituiert sind), für Aminosulfonyl, Mono-($C_1$-$C_4$-alkyl)-aminosulfonyl, Di-($C_1$-$C_4$- alkyl)-aminosulfonyl oder $C_1$-$C_4$-Alkoxy-carbonyl oder Dimethylaminocarbonyl stehen;

weiterhin

$R^3$ für den Rest

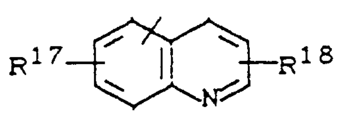

steht, worin

$R^{17}$ und $R^{18}$ gleich oder verschieden sind und für Wasserstoff, Fluor, Chlor, Brom, $C_1$– $C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

weiterhin

$R^3$ für den Rest

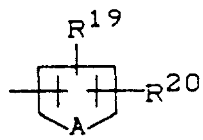

steht, worin

$R^{19}$ und $R^{20}$ gleich oder verschieden sind und für Wasserstoff, Fluor, Chlor, Brom, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/ oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl, $C_1$-$C_4$-Alkoxycarbonyl oder Dimethylaminocarbonyl stehen, und

A für Sauerstoff, Schwefel oder die Gruppierung N-$Z^1$ steht, wobei
$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl Benzyl, Phenyl, (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiterhin

$R^3$ für den Rest

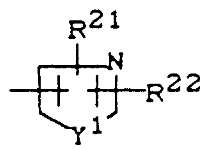

steht, worin

$R^{21}$ und $R^{22}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{23}$ steht, wobei

$R^{23}$ für Wasserstoff oder $C_1$-$C_4$-Alkyl steht;

weiterhin

$R^3$ für den Rest

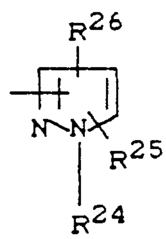

steht, worin

$R^{24}$ für Wasserstoff, $C_1$-$C_4$-Alkyl, Benzyl, Pyridyl, Chinolinyl oder Phenyl steht,

$R^{25}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{26}$ für Wasserstoff, Halogen oder $C_1$-$C_4$-Alkyl steht;

weiterhin

$R^3$ für eine der nachstehend aufgeführten Gruppierungen steht:

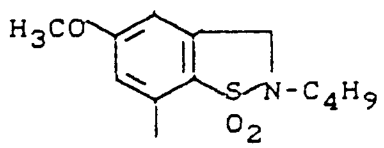 ; 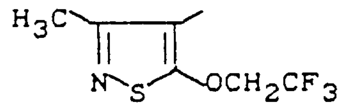

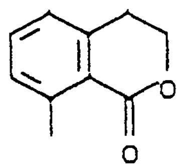

sowie von Salzen von Verbindungen der Formel (I),
dadurch gekennzeichnet, daß man

a) halogenierte Triazolinone der allgemeinen Formel (II)

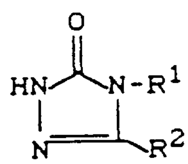 (II)

in welcher
$R^1$ und $R^2$ die oben angegebenen Bedeutungen haben,
mit Sulfonylisocyanaten der allgemeinen Formel (III),

$R^3\text{-}SO_2\text{-}N=C=O$ (III)

in welcher
$R^3$ die oben angegebene Bedeutung hat,
gegebenenfalls in Gegenwart eines Verdünnungsmittels umsetzt, oder daß man

b) halogenierte Triazolinon-Derivate der allgemeinen Formel (IV)

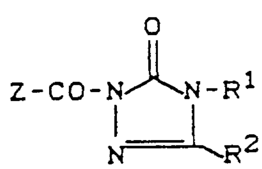 (IV)

in welcher
$R^1$ und $R^2$ die oben angegebenen Bedeutungen haben und
Z für Chlor, $C_1$-$C_4$-Alkoxy, Benzyloxy oder Phenoxy steht,
mit Sulfonsäureamiden der allgemeinen Formel (V),

$R^3\text{-}SO_2\text{-}NH_2$ (V)

in welcher
$R^3$ die oben angegebene Bedeutung hat,
gegebenenfalls in Gegenwart eines Säureakzeptors und gegebenenfalls in Gegenwart eines Verdünnungsmittels umsetzt, oder daß man

c) halogenierte Triazolinone der allgemeinen Formel (II),

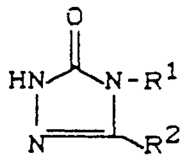

(II)

in welcher
$R^1$ und $R^2$ die oben angegebenen Bedeutungen haben,
mit Sulfonsäureamid-Derivaten der allgemeinen Formel (VI),

$R^3$-$SO_2$-NH-CO-Z (VI)

in welcher
$R^3$ die oben angegebene Bedeutung hat und
Z für Chlor, $C_1$-$C_4$-Alkoxy, Benzyloxy oder Phenoxy steht,
gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

**2.** Verfahren zur Herstellung von halogenierten Sulfonylaminocarbonyltriazolinonen der Formel (I) und von deren Salzen gemäß Anspruch 1, dadurch gekennzeichnet, daß in Formel (I)
$R^1$ für Wasserstoff, für gegebenenfalls durch Fluor, Cyano, Methoxy oder Ethoxy substituiertes $C_1$-$C_4$-Alkyl, für Allyl, für $C_3$-$C_6$-Cycloalkyl, für Phenyl, für $C_1$-$C_4$-Alkoxy, $C_3$-$C_4$-Alkenyloxy, für $C_1$-$C_3$-Alkylamino, $C_3$-$C_6$-Cycloalkylamino oder für Di-($C_1$-$C_3$-alkyl)-amino steht,
$R^2$ für Chlor oder Brom steht, und
$R^3$ für die Gruppierung

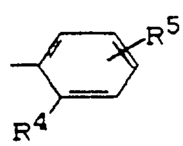

steht, worin
$R^4$ für Fluor, Chlor, Brom, Methyl, Trifluormethyl, Methoxy, Difluormethoxy, Trifluormethoxy, 2-Chlor-ethoxy, 2-Methoxy-ethoxy, $C_1$-$C_3$-Alkylthio, $C_1$-$C_3$-Alkylsulfinyl, $C_1$-$C_3$-Alkyl-sulfonyl, Dimethylaminosulfonyl, Diethylaminosulfonyl, N-Methoxy-N-methylaminosulfonyl, N-Methoxyaminosulfonyl, Phenyl, Phenoxy oder $C_1$-$C_3$-Alkoxy-carbonyl steht und
$R^5$ für Wasserstoff, Fluor, Chlor oder Brom steht;
weiterhin
$R^3$ für den Rest

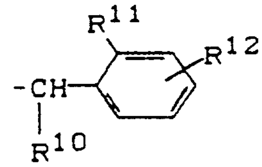

steht, worin
$R^{10}$ für Wasserstoff steht,
$R^{11}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{12}$ für Wasserstoff steht;

weiterhin

$R^3$ für den Rest

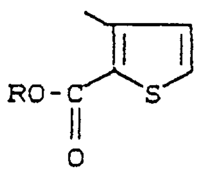

steht,
worin R für $C_1$-$C_4$-Alkyl steht, oder
für den Rest

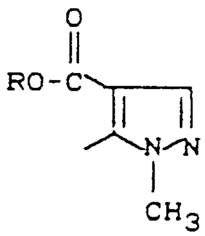

steht,
worin R für $C_1$-$C_4$-Alkyl steht.

3. Herbizide Mittel, gekennzeichnet durch einen Gehalt an mindestens einem halogenierten Sulfonylaminocarbonyltriazolinon der Formel (I) gemaß Anspruch 1.

4. Verwendung von halogenierten Sulfonylaminocarbonyltriazlinonen der allgemeinen Formel (I) gemäß Anspruch 1 zur Bekämpfung von unerwünschtem Pflanzenwachstum.

5. Verfahren zur Bekämpfung von Unkräutern, dadurch gekennzeichnet, daß man halogenierte Sulfonylaminocarbonyltriazolinone der allgemeinen Formel (I) gemäß Anspruch 1 auf die Unkräuter oder ihren Lebensraum einwirken läßt.

6. Verfahren zur Herstellung von herbiziden Mitteln, dadurch gekennzeichnet, daß man halogenierte Sulfonylaminocarbonyltriazolinone der allgemeinen Formel (I) gemäß Anspruch 1 mit Streckmitteln und/oder oberflächenaktiven Mitteln vermischt.

7. Verfahren zur Herstellung von halogenierten Triazolinonen der Formel (IV),

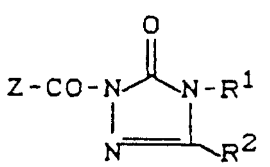

(IV)

in welcher

$R^1$ und $R^2$ die in Anspruch 1 angegebenen Bedeutungen haben und

Z für Chlor, $C_1$-$C_4$-Alkoxy, Benzyloxy oder Phenoxy steht,

dadurch gekennzeichnet, daß man halogenierte Triazolinone der Formel (II),

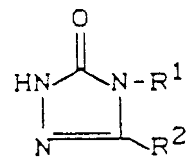

(II)

in welcher
$R^1$ und $R^2$ die oben angegebenen Bedeutungen haben,
mit Phosgen, Chlorameisensäure-methylester, Chlorameisensäure-benzylester, Chlorameisensäure-phenylester oder Diphenylcarbonat umsetzt.

**Claims**

**Claims for the following Contracting States : BE, CH, DE, DK, FR, GB, IT, LI, NL**

**1.** Halogenated sulphonylaminocarbonyltriazolinones of the general formula (I)

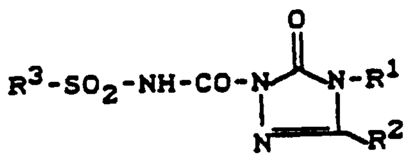

(I)

in which

$R^1$ represents hydrogen, hydroxyl or amino, or represents $C_1$-$C_6$-alkyl which is optionally substituted by fluorine, chlorine, bromine, cyano, $C_1$-$C_4$-alkoxy, $C_1$-$C_4$-alkylcarbonyl or $C_1$-$C_4$-alkoxy-carbonyl, or represents $C_3$-$C_6$-alkenyl or $C_3$-$C_6$-alkinyl, in each case optionally substituted by fluorine, chlorine and/or bromine, or represents $C_3$-$C_6$-cycloalkyl which is optionally substituted by fluorine, chlorine, bromine and/or $C_1$-$C_4$-alkyl, or represents phenyl-$C_1$-$C_3$-alkyl which is optionally substituted by fluorine, chlorine, bromine, cyano, nitro, $C_1$-$C_4$-alkyl, trifluoromethyl, $C_1$-$C_4$-alkoxy and/or $C_1$-$C_4$-alkoxy-carbonyl, or represents phenyl which is optionally substituted by fluorine, chlorine, bromine, cyano, nitro, $C_1$-$C_4$-alkyl, trifluoromethyl, $C_1$-$C_4$-alkoxy, fluorine- and/or chlorine-substituted $C_1$-$C_3$-alkoxy, $C_1$-$C_4$-alkylthio, fluorine- and/or chlorine-substituted $C_1$-$C_3$-alkylthio, $C_1$-$C_4$-alkylsulphinyl, $C_1$-$C_4$-alkylsulphonyl and/or $C_1$-$C_4$-alkoxy-carbonyl, or represents $C_1$-$C_6$-alkoxy which is optionally substituted by fluorine, chlorine, cyano, $C_1$-$C_4$-alkoxy or $C_1$-$C_4$-alkoxy-carbonyl, or represents $C_3$-$C_4$-alkenyloxy, or represents $C_1$-$C_4$-alkylamino which is optionally substituted by fluorine, cyano, phenyl, $C_1$-$C_4$-alkoxy or $C_1$-$C_4$-alkoxy-carbonyl, $C_3$-$C_6$-cycloalkylamino or represents di-($C_1$-$C_4$-alkyl)-amino,

$R^2$ represents fluorine, chlorine, bromine or iodine and

$R^3$ represents the grouping

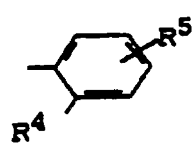

wherein
$R^4$ and $R^5$ are identical or different and represent hydrogen, fluorine, chlorine, bromine, iodine, nitro, $C_1$-$C_6$-alkyl (which is optionally substituted by fluorine, chlorine, bromine, cyano, carboxyl, $C_1$-$C_4$-alkoxycarbonyl, $C_1$-$C_4$-alkylamino-carbonyl di-($C_1$-$C_4$-alkyl)amino-carbonyl, hydroxyl, $C_1$ -$C_4$-alkoxy, formyloxy, $C_1$-$C_4$-alkyl-carbonyloxy, $C_1$-$C_4$-alkoxy-carbonyloxy, $C_1$-$C_4$-alkylamino-carbonyloxy, $C_1$-

$C_4$-alkylthio, $C_1$-$C_4$-alkylsulphinyl, $C_1$-$C_4$-alkylsulphonyl, di-($C_1$-$C_4$-alkyl)-aminosulphonyl, $C_3$-$C_6$-cycloalkyl or phenyl), or represent $C_2$-$C_6$-alkenyl (which is optionally substituted by fluorine, chlorine, bromine, cyano, $C_1$-$C_4$-alkoxy-carbonyl, carboxyl or phenyl), or represent $C_2$-$C_6$-alkinyl (which is optionally substituted by fluorine, chlorine, bromine, cyano, $C_1$-$C_4$-alkoxy-carbonyl, carboxyl or phenyl), or represent $C_1$-$C_4$-alkoxy (which is optionally substituted by fluorine, chlorine, bromine, cyano, carboxyl, $C_1$-$C_4$-alkoxy-carbonyl, $C_1$-$C_4$-alkoxy, $C_1$-$C_4$-alkylthio, $C_1$-$C_4$-alkylsulphinyl or $C_1$-$C_4$-alkylsulphonyl), or represent $C_1$-$C_4$-alkylthio (which is optionally substituted by fluorine, chlorine, bromine, cyano, carboxyl, $C_1$-$C_4$-alkoxy-carbonyl, $C_1$-$C_4$-alkylthio, $C_1$-$C_4$alkylsulphinyl or $C_1$-$C_4$-alkylsulphonyl), or represent $C_3$-$C_6$-alkenyloxy (which is optionally substituted by fluorine, chlorine, bromine, cyano or $C_1$-$C_4$-alkoxycarbonyl), or represent $C_2$-$C_6$-alkenylthio which is optionally substituted by fluorine, chlorine, bromine, cyano, nitro, $C_1$-$C_3$-alkylthio or $C_1$-$C_4$-alkoxycarbonyl), $C_3$-$C_6$-alkinyloxy, $C_3$-$C_6$-alkinylthio or represent the radical $-S(O)_p-R^6$, wherein

p represents the numbers 1 or 2 and

$R^6$ represents $C_1$-$C_4$-alkyl (which is optionally substituted by fluorine, chlorine, bromine, cyano or $C_1$-$C_4$-alkoxy-carbonyl),$C_3$-$C_6$-alkenyl, $C_3$-$C_6$-alkinyl, $C_1$-$C_4$-alkoxy,$C_1$-$C_4$-alkoxy-$C_1$-$C_4$-alkylamino, $C_1$-$C_4$-alkylamino, di-($C_1$-$C_4$-alkyl)-amino, phenyl or represents the radical $-NHOR^7$, wherein

$R^7$ represents $C_1$-$C_{12}$-alkyl (which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkylcarbonyl, $C_1$-$C_4$-alkoxycarbonyl, $C_1$-$C_4$-alkylamino-carbonyl or di-($C_1$-$C_4$-alkyl)-amino-carbonyl), or represents $C_3$-$C_6$-alkenyl (which is optionally substituted by fluorine, chlorine or bromine), $C_3$-$C_6$-alkinyl, $C_3$-$C_6$-cycloalkyl, $C_3$-$C_6$-cycloalkyl-$C_1$-$C_2$-alkyl, phenyl-$C_1$-$C_2$-alkyl (which is optionally substituted by fluorine, chlorine, nitro, cyano, $C_1$-$C_4$-alkyl, $C_1$-$C_4$-alkoxy or $C_1$-$C_4$-alkoxy-carbonyl), or represents benzhydryl or represents phenyl (which is optionally substituted by fluorine, chlorine, nitro, cyano, $C_1$-$C_4$-alkyl, trifluoromethyl, $C_1$-$C_4$-alkoxy, $C_1$-$C_2$-fluoroalkoxy, $C_1$-$C_4$-alkylthio, trifluoromethylthio or $C_1$-$C_4$-alkoxy-carbonyl),

$R^4$ and/or $R^5$ furthermore represent phenyl or phenoxy, or represent $C_1$-$C_4$-alkylcarbonylamino, $C_1$-$C_4$-alkoxycarbonylamino, $C_1$-$C_4$-alkylamino-carbonyl-amino, di-($C_1$-$C_4$-alkyl)-amino-carbonylamino, or represent the radical $-CO-R^8$, wherein

$R^8$ represents $C_1$-$C_6$-alkyl, $C_1$-$C_6$-alkoxy, $C_3$-$C_6$-cycloalkoxy, $C_3$-$C_6$-alkenyloxy, $C_1$-$C_4$-alkylthio, $C_1$-$C_4$-alkylamino, $C_1$-$C_4$-alkoxyamino, $C_1$-$C_4$-alkoxy-$C_1$-$C_4$-alkyl-amino or di-($C_1$-$C_4$-alkyl)-amino (which are optionally substituted by fluorine and/or chlorine),

$R^4$ and/or $R^5$ furthermore represent trimethylsilyl, thiazolinyl, $C_1$-$C_4$-alkylsulphonyloxy, di-($C_1$-$C_4$-alkyl)-aminosulphonylamino or represent the radical $-CH=N-R^9$, wherein

$R^9$ represents $C_1$-$C_6$-alkyl which is optionally substituted by fluorine, chlorine, cyano, carboxyl, $C_1$-$C_4$-alkoxy, $C_1$-$C_4$-alkylthio, $C_1$-$C_4$-alkylsulphinyl or $C_1$-$C_4$-alkylsulphonyl, or represents benzyl which is optionally substituted by fluorine or chlorine, or represents $C_3$-$C_6$-alkenyl or $C_3$-$C_6$-alkinyl which are optionally substituted by fluorine or chlorine, or represents phenyl which is optionally substituted by fluorine, chlorine, bromine, $C_1$-$C_4$-alkyl, $C_1$-$C_4$-alkoxy, trifluoromethyl, trifluoromethoxy or trifluoromethylthio, or represents $C_1$-$C_6$-alkoxy, $C_3$-$C_6$-alkenoxy, $C_3$-$C_6$-alkinoxy or benzyloxy which are optionally substituted by fluorine and/or chlorine or represents amino, $C_1$-$C_4$-alkylamino, di-($C_1$-$C_4$-alkyl)-amino, phenylamino, $C_1$-$C_4$-alkylcarbonyl-amino, $C_1$-$C_4$-alkoxy-carbonylamino, $C_1$-$C_4$-alkyl-sulphonylamino or represents phenylsulphonylamino which is optionally substituted by fluorine, chlorine, bromine or methyl,

or furthermore

$R^3$ represents the radical

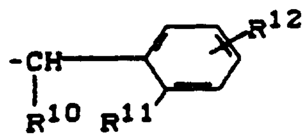

wherein

$R^{10}$ represents hydrogen or $C_1$-$C_4$-alkyl,

$R^{11}$ and $R^{12}$ are identical or different and represent hydrogen, fluorine, chlorine, bromine, nitro, cyano, $C_1$-$C_4$-alkyl (which is optionally substituted by fluorine and/or chlorine), $C_1$-$C_4$-alkoxy (which is optionally substituted by fluorine and/or chlorine), carboxyl, $C_1$-$C_4$-alkoxy-carbonyl, dimethylaminocarbonyl, $C_1$-$C_4$-alkylsulphonyl or di-($C_1$-$C_4$-alkyl)-aminosulphonyl;

or furthermore

$R^3$ represents the radical

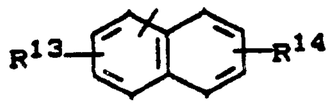

wherein

$R^{13}$ and $R^{14}$ are identical or different and represent hydrogen, fluorine, chlorine, bromine, nitro, cyano, $C_1$-$C_4$-alkyl (which is optionally substituted by fluorine and/or chlorine) or $C_1$-$C_4$-alkoxy (which is optionally substituted by fluorine and/or chlorine);

or furthermore

$R^3$ represents the radical

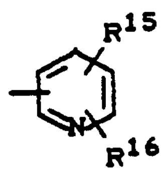

wherein

$R^{15}$ and $R^{16}$ are identical or different and represent hydrogen, fluorine, chlorine, bromine, nitro, cyano, $C_1$-$C_4$-alkyl (which is optionally substituted by fluorine and/or chlorine), $C_1$-$C_4$-alkoxy (which is optionally substituted by fluorine and/or chlorine), or represent $C_1$-$C_4$-alkylthio, $C_1$-$C_4$-alkylsulphinyl or $C_1$-$C_4$-alkylsulphonyl (which are optionally substituted by fluorine and/or chlorine), or represent aminosulphonyl, mono-($C_1$-$C_4$-alkyl)-aminosulphonyl, di-($C_1$-$C_4$-alkyl)-aminosulphonyl or $C_1$-$C_4$-alkoxy-carbonyl or dimethylaminocarbonyl;

or furthermore

$R^3$ represents the radical

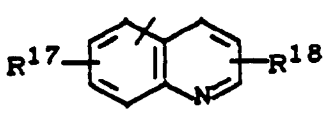

wherein

$R^{17}$ and $R^{18}$ are identical or different and represent hydrogen, fluorine, chlorine, bromine, $C_1$-$C_4$-alkyl (which is optionally substituted by fluorine and/or bromine), $C_1$-$C_4$-alkoxy (which is optionally substituted by fluorine and/or chlorine), or represent $C_1$-$C_4$-

alkylthio, $C_1$-$C_4$-alkylsulphinyl or $C_1$-$C_4$-alkylsulphonyl (which are optionally substituted by fluorine and/or chlorine), or represent di-($C_1$-$C_4$-alkyl)-aminosulphonyl;

or furthermore

$R^3$ represents the radical

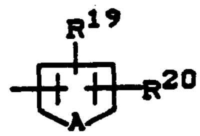

wherein

$R^{19}$ and $R^{20}$ are identical or different and represent hydrogen, fluorine, chlorine, bromine, cyano, nitro, $C_1$-$C_4$-alkyl (which is optionally substituted by fluorine and/or chlorine), $C_1$-$C_4$-alkoxy (which is optionally substituted by fluorine and/or chlorine), $C_1$-$C_4$-alkylthio, $C_1$-$C_4$-alkylsulphinyl or $C_1$-$C_4$-alkylsulphonyl (which is optionally substituted by fluorine and/or chlorine), di-($C_1$-$C_4$-alkyl)-aminosulphonyl, $C_1$-$C_4$-alkoxy-carbonyl or dimethylaminocarbonyl, and

A represents oxygen, sulphur or the grouping N-$Z^1$, wherein

$Z^1$ represents hydrogen, $C_1$-$C_4$-alkyl (which is optionally substituted by fluorine, chlorine, bromine or cyano), $C_3$-$C_6$-cycloalkyl, benzyl, phenyl, (which is optionally substituted by fluorine, chlorine, bromine or nitro), $C_1$–$C_4$-alkylcarbonyl, $C_1$–$C_4$–alkoxy-carbonyl or di-($C_1$-$C_4$-alkyl)-aminocarbonyl;

or furthermore

$R^3$ represents the radical

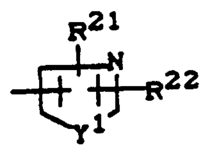

wherein

$R^{21}$ and $R^{22}$ are identical or different and represent hydrogen, $C_1$-$C_4$-alkyl, halogen, $C_1$-$C_4$-alkoxycarbonyl, $C_1$-$C_4$-alkoxy or $C_1$-$C_4$-halogenoalkoxy,

$Y^1$ represents sulphur or the grouping N-$R^{23}$, wherein

$R^{23}$ represents hydrogen or $C_1$-$C_4$-alkyl;

or furthermore

$R^3$ represents the radical

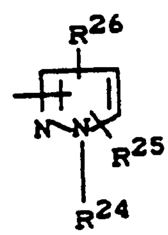

wherein

$R^{24}$ represents hydrogen, $C_1$-$C_4$-alkyl, benzyl, pyridyl, quinolinyl or phenyl,

$R^{25}$ represents hydrogen, halogen, cyano, nitro, $C_1$-$C_4$-alkyl (which is optionally substituted by fluorine and/or chlorine), $C_1$-$C_4$-alkoxy (which is optionally substituted by fluorine and/or chlorine), dioxolanyl or $C_1$-$C_4$-alkoxy-carbonyl and

$R^{26}$ represents hydrogen, halogen or $C_1$-$C_4$-alkyl;

or furthermore

$R^3$ represents one of the groupings listed below:

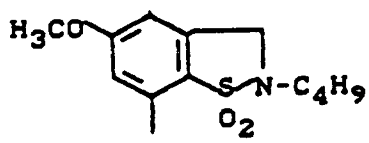 ; 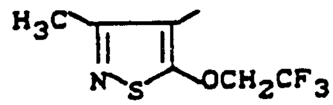

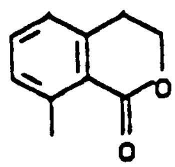

and salts of compounds of the formula (I).

2. Halogenated sulphonylaminocarbonyltriazolinones of the general formula (I) according to Claim 1, in which
   - $R^1$ represents hydrogen, or represents $C_1$-$C_4$-alkyl which is optionally substituted by fluorine, cyano, methoxy or ethoxy, or represents allyl, or represents $C_3$-$C_6$-cycloalkyl, or represents phenyl, or represents $C_1$-$C_4$-alkoxy, $C_3$-$C_4$-alkenyloxy, or represents $C_1$-$C_3$-alkylamino, $C_3$-$C_6$-cycloalkylamino or represents di-($C_1$–$C_3$-alkyl)-amino,
   - $R^2$ represents chlorine or bromine and
   - $R^3$ represents the grouping

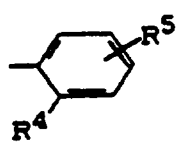

wherein
- $R^4$ represents fluorine, chlorine, bromine, methyl, trifluoromethyl, methoxy, difluoromethoxy, trifluoromethoxy, 2-chloroethoxy, 2-methoxyethoxy, $C_1$-$C_3$-alkylthio, $C_1$-$C_3$-alkylsulphinyl, $C_1$-$C_3$-alkylsulphonyl, dimethylaminosulphonyl, diethylaminosulphonyl, N-methoxy-N-methylaminosulphonyl, N-methoxyaminosulphonyl, phenyl, phenoxy or $C_1$-$C_3$-alkoxy-carbonyl and
- $R^5$ represents hydrogen, fluorine, chlorine or bromine;

or furthermore
- $R^3$ represents the radical

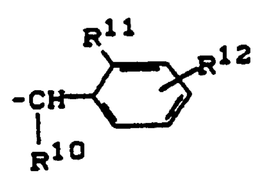

wherein
- $R^{10}$ represents hydrogen,
- $R^{11}$ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
- $R^{12}$ represents hydrogen;

or furthermore
- $R^3$ represents the radical

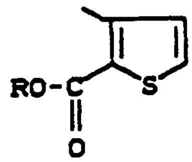

wherein R represents $C_1$-$C_4$-alkyl, or
represents the radical

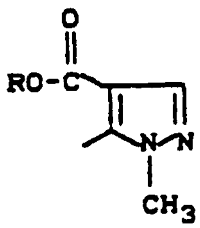

wherein R represents $C_1$-$C_4$-alkyl,
and salts of compounds of the formula (I).

**3.** Process for the preparation of halogenated sulphonylaminocarbonyltriazolinones of the general formula (I) according to Claim 1 and of salts of compounds of the formula (I),
characterized in that
a) halogenated triazolinones of the general formula (II)

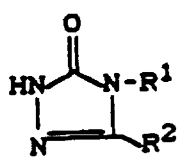

(II)

in which
$R^1$ and $R^2$ have the meanings given in Claim 1,
are reacted with sulphonyl isocyanates of the general formula (III)

$R^3$-$SO_2$-N = C = O (III)

in which
$R^3$ has the meaning given in Claim 1,
if appropriate in the presence of a diluent, or in that
b) halogenated triazolinone derivatives of the general formula (IV)

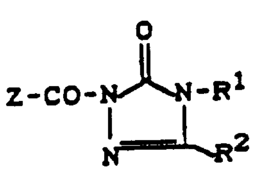

(IV)

in which
$R^1$ and $R^2$ have the meanings given in Claim 1 and
Z represents chlorine, $C_1$-$C_4$-alkoxy, benzyloxy or phenoxy,
are reacted with sulphonic acid amides of the general formula (V),

$R^3$-$SO_2$-$NH_2$ (V)

in which

$R^3$ has the meaning given in Claim 1,

if appropriate in the presence of an acid acceptor and if appropriate in the presence of a diluent, or in that

c) halogenated triazolinones of the general formula (II)

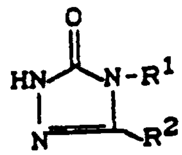

(II)

in which

$R^1$ and $R^2$ have the meanings given in Claim 1,

are reacted with sulphonic acid amide derivatives of the general formula (VI),

$R^3$-$SO_2$-NH-CO-Z (VI)

in which

$R^3$ has the meaning given in Claim 1 and

Z represents chlorine, $C_1$-$C_4$-alkoxy, benzyloxy or phenoxy,

if appropriate in the presence of an acid acceptor and if appropriate in the presence of a diluent, and if appropriate salts are obtained by customary methods from the compounds of the formula (I) prepared according to process (a), (b) or (c).

**4.** Herbicidal compositions, characterized in that they contain at least one halogenated sulphonylaminocarbonyltriazolinone of the formula (I) according to Claim 1.

**5.** Use of halogenated sulphonylaminocarbonyltriazolinones of the general formula (I) according to Claim 1 for combating undesirable plant growth.

**6.** Method of combating weeds, characterized in that halogenated sulphonylaminocarbonyltriazolinones of the general formula (I) according to Claim 1 are allowed to act on the weeds or their environment.

**7.** Process for the preparation of herbicidal compositions, characterized in that halogenated sulphonylaminocarbonyltriazolinones of the general formula (I) according to Claim 1 are mixed with extenders and/or surface-active agents.

**8.** Halogenated triazolinones of the general formula (IV)

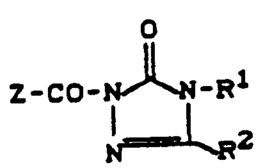

(IV)

in which

$R^1$ and $R^2$ have the meanings given in Claim 1 and

Z represents chlorine, $C_1$-$C_4$-alkoxy, benzyloxy or phenoxy.

**Claims for the following Contracting State : ES**

**1.** Process for the preparation of halogenated sulphonylaminocarbonyltriazolinones of the general formula (I)

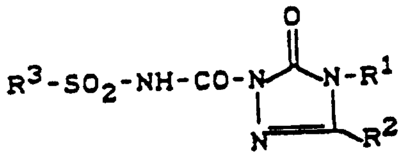

(I)

in which

$R^1$ represents hydrogen, hydroxyl or amino, or represents $C_1$-$C_6$-alkyl which is optionally substituted by fluorine, chlorine, bromine, cyano, $C_1$-$C_4$-alkoxy, $C_1$-$C_4$-alkylcarbonyl or $C_1$-$C_4$-alkoxy-carbonyl, or represents $C_3$-$C_6$-alkenyl or $C_3$-$C_6$-alkinyl, in each case optionally substituted by fluorine, chlorine and/or bromine, or represents $C_3$-$C_6$-cycloalkyl which is optionally substituted by fluorine, chlorine, bromine and/or $C_1$-$C_4$-alkyl, or represents phenyl $C_1$-$C_3$-alkyl which is optionally substituted by fluorine, chlorine, bromine, cyano, nitro, $C_1$-$C_4$-alkyl, trifluoromethyl, $C_1$-$C_4$-alkoxy and/or $C_1$-$C_4$-alkoxy-carbonyl, or represents phenyl which is optionally substituted by fluorine, chlorine, bromine, cyano, nitro, $C_1$-$C_4$-alkyl, trifluoromethyl, $C_1$-$C_4$-alkoxy, fluorine- and/or chlorine-substituted $C_1$-$C_3$-alkoxy, $C_1$-$C_4$-alkylthio, fluorine- and/or chlorine-substituted $C_1$-$C_3$-alkylthio, $C_1$-$C_4$-alkylsulphinyl, $C_1$-$C_4$-alkylsulphonyl and/or $C_1$-$C_4$-alkoxy-carbonyl, or represents $C_1$-$C_6$-alkoxy which is optionally substituted by fluorine, chlorine, cyano, $C_1$-$C_4$-alkoxy or $C_1$-$C_4$-alkoxy-carbonyl, or represents $C_3$-$C_4$-alkenyloxy, or represents $C_1$-$C_4$-alkylamino which is optionally substituted by fluorine, cyano, phenyl, $C_1$-$C_4$-alkoxy or $C_1$-$C_4$-alkoxy-carbonyl, $C_3$-$C_6$-cycloalkylamino or represents di-($C_1$-$C_4$-alkyl)-amino,

$R^2$ represents fluorine, chlorine, bromine or iodine and

$R^3$ represents the grouping

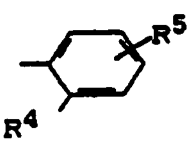

wherein

$R^4$ and $R^5$ are identical or different and represent hydrogen, fluorine, chlorine, bromine, iodine, nitro, $C_1$-$C_6$-alkyl (which is optionally substituted by fluorine, chlorine, bromine, cyano, carboxyl, $C_1$-$C_4$-alkoxycarbonyl, $C_1$-$C_4$-alkylamino-carbonyl, di-($C_1$-$C_4$-alkyl)amino-carbonyl, hydroxyl, $C_1$-$C_4$-alkoxy, formyloxy, $C_1$-$C_4$-alkyl-carbonyloxy, $C_1$-$C_4$-alkoxy-carbonyloxy, $C_1$-$C_4$-alkylamino-carbonyloxy, $C_1$-$C_4$-alkylthio, $C_1$-$C_4$-alkylsulphinyl, $C_1$-$C_4$-alkylsulphonyl, di-($C_1$-$C_4$-alkyl)-aminosulphonyl, $C_3$-$C_6$-cycloalkyl or phenyl), or represent $C_2$-$C_6$-alkenyl (which is optionally substituted by fluorine, chlorine, bromine, cyano, $C_1$-$C_4$-alkoxy-carbonyl, carboxyl or phenyl), or represent $C_2$-$C_6$-alkinyl (which is optionally substituted by fluorine, chlorine, bromine, cyano, $C_1$-$C_4$-alkoxy-carbonyl, carboxyl or phenyl), or represent $C_1$-$C_4$-alkoxy (which is optionally substituted by fluorine, chlorine, bromine, cyano, carboxyl, $C_1$-$C_4$-alkoxy-carbonyl, $C_1$-$C_4$-alkoxy, $C_1$-$C_4$-alkylthio, $C_1$-$C_4$-alkylsulphinyl or $C_1$-$C_4$-alkylsulphonyl), or represent $C_1$-$C_4$-alkylthio (which is optionally substituted by fluorine, chlorine, bromine, cyano, carboxyl, $C_1$-$C_4$-alkoxy-carbonyl, $C_1$-$C_4$-alkylthio, $C_1$-$C_4$-alkylsulphinyl or $C_1$-$C_4$-alkylsulphonyl), or represent $C_3$-$C_6$-alkenyloxy (which is optionally substituted by fluorine, chlorine, bromine, cyano or $C_1$-$C_4$-alkoxycarbonyl), or represent $C_2$-$C_6$-alkenylthio which is optionally substituted by fluorine, chlorine, bromine, cyano, nitro, $C_1$-$C_3$-alkylthio or $C_1$-$C_4$-alkoxycar-

bonyl), $C_3$-$C_6$-alkinyloxy, $C_3$-$C_6$-alkinylthio or represent the radical -S(O)$_p$-$R^6$, wherein

p represents the numbers 1 or 2 and

$R^6$ represents $C_1$-$C_4$-alkyl (which is optionally substituted by fluorine, chlorine, bromine, cyano or $C_1$-$C_4$-alkoxy-carbonyl),$C_3$-$C_6$-alkenyl, $C_3$-$C_6$-alkinyl, $C_1$-$C_4$-alkoxy, $C_1$-$C_4$-alkoxy-$C_1$-$C_4$-alkylamino, $C_1$-$C_4$-alkylamino, di-($C_1$-$C_4$-alkyl)-amino, phenyl or represents the radical -NHOR$^7$, wherein

$R^7$ represents $C_1$-$C_{12}$-alkyl (which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkylcarbonyl, $C_1$-$C_4$-alkoxycarbonyl, $C_1$-$C_4$-alkylamino-carbonyl or di-($C_1$-$C_4$-alkyl) amino-carbonyl), or represents $C_3$-$C_6$-alkenyl (which is optionally substituted by fluorine, chlorine or bromine), $C_3$-$C_6$-alkinyl, $C_3$-$C_6$-cycloalkyl, $C_3$-$C_6$-cycloalkyl-$C_1$-$C_2$-alkyl, phenyl-$C_1$-$C_2$-alkyl (which is optionally substituted by fluorine, chlorine, nitro, cyano, $C_1$-$C_4$-alkyl, $C_1$-$C_4$-alkoxy or $C_1$-$C_4$-alkoxy-carbonyl), or represents benzhydryl or represents phenyl (which is optionally substituted by fluorine, chlorine, nitro, cyano, $C_1$-$C_4$-alkyl, trifluoromethyl, $C_1$-$C_4$-alkoxy, $C_1$-$C_2$-fluoroalkoxy, $C_1$-$C_4$-alkylthio, trifluoromethylthio or $C_1$-$C_4$-alkoxy-carbonyl),

$R^4$ and/or $R^5$ furthermore represent phenyl or phenoxy, or represent $C_1$-$C_4$-alkylcarbonyl amino, $C_1$-$C_4$-alkoxycarbonylamino, $C_1$-$C_4$-alkylamino-carbonyl-amino, di-($C_1$-$C_4$-alkyl)-amino-carbonylamino, or represent the radical -CO-$R^8$, wherein

$R^8$ represents $C_1$-$C_6$-alkyl, $C_1$-$C_6$-alkoxy, $C_3$-$C_6$-cycloalkoxy, $C_3$-$C_6$-alkenyloxy, $C_1$-$C_4$-alkylthio, $C_1$-$C_4$-alkylamino, $C_1$-$C_4$-alkoxyamino, $C_1$-$C_4$-alkoxy-$C_1$-$C_4$-alkyl-amino or di($C_1$-$C_4$-alkyl)-amino (which are optionally substituted by fluorine and/or chlorine),

$R^4$ and/or $R^5$ furthermore represent trimethylsilyl, thiazolinyl, $C_1$-$C_4$-alkylsulphonyloxy, di-($C_1$-$C_4$-alkyl)-aminosulphonylamino or represent the radical -CH=N-$R^9$, wherein

$R^9$ represents $C_1$-$C_6$-alkyl which is optionally substituted by fluorine, chlorine, cyano, carboxyl, $C_1$-$C_4$-alkoxy, $C_1$-$C_4$-alkylthio, $C_1$-$C_4$-alkylsulphinyl or $C_1$-$C_4$-alkylsulphonyl, or represents benzyl which is optionally substituted by fluorine or chlorine, or represents $C_3$-$C_6$-alkenyl or $C_3$-$C_6$-alkinyl which are optionally substituted by fluorine or chlorine, or represents phenyl which is optionally substituted by fluorine, chlorine, bromine, $C_1$-$C_4$-alkyl, $C_1$-$C_4$-alkoxy, trifluoromethyl, trifluoromethoxy or trifluoromethylthio, or represents $C_1$-$C_6$-alkoxy, $C_3$-$C_6$-alkenoxy, $C_3$-$C_6$-alkinoxy or benzyloxy which are optionally substituted by fluorine and/or chlorine or represents amino, $C_1$-$C_4$-alkylamino, di-($C_1$-$C_4$-alkyl)-amino, phenylamino, $C_1$-$C_4$-alkyl-carbonyl-amino, $C_1$-$C_4$-alkoxy-carbonylamino, $C_1$-$C_4$-alkylsulphonylamino or represents phenylsulphonylamino which is optionally substituted by fluorine, chlorine,

bromine or methyl, or furthermore

$R^3$ represents the radical

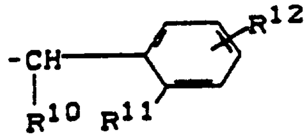

wherein

$R^{10}$ represents hydrogen or $C_1$-$C_4$-alkyl,

$R^{11}$ and $R^{12}$ are identical or different and represent hydrogen, fluorine, chlorine, bromine, nitro, cyano, $C_1$-$C_4$-alkyl (which is optionally substituted by fluorine and/or chlorine), $C_1$-$C_4$-alkoxy (which is optionally substituted by fluorine and/or chlorine), carboxyl, $C_1$-$C_4$-alkoxy-carbonyl, dimethylaminocarbonyl, $C_1$-$C_4$-alkylsulphonyl or di-($C_1$-$C_4$-alkyl)-aminosulphonyl;

or furthermore

$R^3$ represents the radical

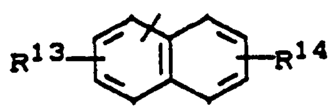

wherein

$R^{13}$ and $R^{14}$ are identical or different and represent hydrogen, fluorine, chlorine, bromine, nitro, cyano, $C_1$-$C_4$-alkyl (which is optionally substituted by fluorine and/or chlorine) or $C_1$-$C_4$-alkoxy (which is optionally substituted by fluorine and/or chlorine);

or furthermore

$R^3$ represents the radical

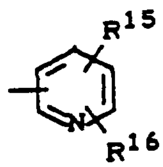

wherein

$R^{15}$ and $R^{16}$ are identical or different and represent hydrogen, fluorine, chlorine, bromine, nitro, cyano, $C_1$-$C_4$-alkyl (which is optionally substituted by fluorine and/or chlorine), $C_1$-$C_4$-alkoxy (which is optionally substituted by fluorine and/or chlorine), or represent $C_1$-$C_4$-alkylthio, $C_1$-$C_4$-alkylsulphinyl or $C_1$-$C_4$-alkylsulphonyl (which are optionally substituted by fluorine and/or chlorine), or represent aminosulphonyl, mono-($C_1$-$C_4$-alkyl)-aminosulphonyl, di-($C_1$-$C_4$-alkyl)-aminosulphonyl or $C_1$-$C_4$-alkoxy-carbonyl or dimethylaminocarbonyl;

or furthermore

$R^3$ represents the radical

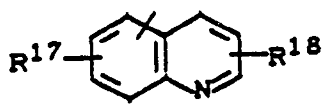

wherein

$R^{17}$ and $R^{18}$ are identical or different and represent hydrogen, fluorine, chlorine, bromine, $C_1$-$C_4$-alkyl (which is optionally substituted by fluorine and/or bromine), $C_1$-$C_4$-alkoxy (which is optionally substituted by fluorine and/or chlorine), or represent $C_1$-$C_4$-alkylthio, $C_1$-$C_4$-alkylsulphinyl or $C_1$-$C_4$-alkylsulphonyl (which are optionally substituted by fluorine and/or chlorine), or represent di-($C_1$-$C_4$-alkyl)-aminosulphonyl;

or furthermore

$R^3$ represents the radical

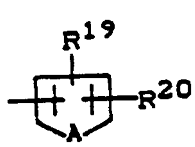

wherein

$R^{19}$ and $R^{20}$ are identical or different and represent hydrogen, fluorine, chlorine, bromine, cyano, nitro, $C_1$-$C_4$-alkyl (which is optionally substituted by fluorine and/or chlorine), $C_1$-$C_4$-

alkoxy (which is optionally substituted by fluorine and/or chlorine), $C_1$-$C_4$-alkylthio, $C_1$-$C_4$-alkylsulphinyl or $C_1$-$C_4$-alkylsulphonyl (which is optionally substituted by fluorine and/or chlorine), di-($C_1$-$C_4$-alkyl)-aminosulphonyl, $C_1$-$C_4$-alkoxy-carbonyl or dimethylaminocarbonyl, and

A represents oxygen, sulphur or the grouping N-$Z^1$, wherein

$Z^1$ represents hydrogen, $C_1$-$C_4$-alkyl (which is optionally substituted by fluorine, chlorine, bromine or cyano), $C_3$-$C_6$-cycloalkyl, benzyl, phenyl, (which is optionally substituted by fluorine, chlorine, bromine or nitro), $C_1$-$C_4$-alkylcarbonyl, $C_1$-$C_4$-alkoxy-carbonyl or di-($C_1$-$C_4$-alkyl)-aminocarbonyl;

or furthermore

$R^3$ represents the radical

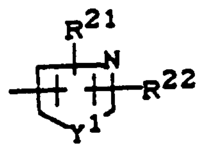

wherein

$R^{21}$ and $R^{22}$ are identical or different and represent hydrogen, $C_1$-$C_4$-alkyl, halogen, $C_1$-$C_4$-alkoxycarbonyl, $C_1$-$C_4$-alkoxy or $C_1$-$C_4$-halogenoalkoxy,

$Y^1$ represents sulphur or the grouping N-$R^{23}$, wherein

$R^{23}$ represents hydrogen or $C_1$-$C_4$-alkyl;

or furthermore

$R^3$ represents the radical

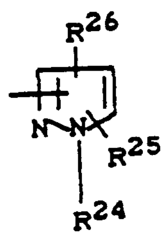

wherein

$R^{24}$ represents hydrogen, $C_1$-$C_4$-alkyl, benzyl, pyridyl, quinolinyl or phenyl,

$R^{25}$ represents hydrogen, halogen, cyano, nitro, $C_1$-$C_4$-alkyl (which is optionally substituted by fluorine and/or chlorine), $C_1$-$C_4$-alkoxy (which is optionally substituted by fluorine and/or chlorine), dioxolanyl or $C_1$-$C_4$-alkoxy-carbonyl and

$R^{26}$ represents hydrogen, halogen or $C_1$-$C_4$-alkyl;

or furthermore

$R^3$ represents one of the groupings listed below:

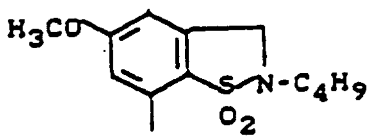

;

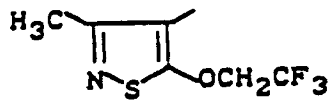

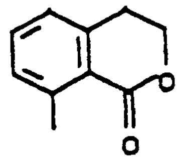

and of salts of compounds of the formula (I),
characterized in that

a) halogenated triazolinones of the general formula (II)

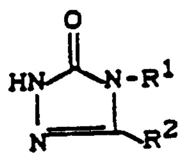

(II)

in which
$R^1$ and $R^2$ have the meanings given above,
are reacted with sulphonyl isocyanates of the general formula (III)

$R^3$-$SO_2$-N = C = O (III)

in which
$R^3$ has the meaning given above,
if appropriate in the presence of a diluent, or in that

b) halogenated triazolinone derivative of the general formula (IV)

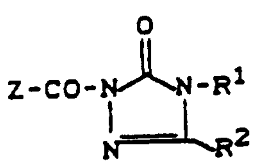

(IV)

in which
$R^1$ and $R^2$ have the meanings given above and
Z represents chlorine, $C_1$-$C_4$-alkoxy, benzyloxy or phenoxy,
are reacted with sulphonic acid amides of the general formula (V),

$R^3$-$SO_2$-$NH_2$ (V)

in which
$R^3$ has the meaning given above,
if appropriate in the presence of an acid acceptor and if appropriate in the presence of a diluent, or in that

c) halogenated triazolinones of the general formula (II)

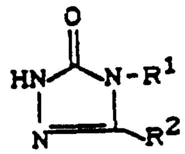

(II)

in which
$R^1$ and $R^2$ have the meanings given above,
are reacted with sulphonic acid amide derivatives of the general formula (VI),

$R^3$-$SO_2$-NH-CO-Z (VI)

in which
$R^3$ has the meaning given above and
Z represents chlorine, $C_1$-$C_4$-alkoxy, benzyloxy or phenoxy,
if appropriate in the presence of an acid acceptor and if appropriate in the presence of a diluent, and if appropriate salts are obtained by customary methods from the compounds of the formula (I) prepared according to process (a), (b) or (c).

**2.** Process for the preparation of halogenated sulphonylaminocarbonyltriazolinones of the formula (I) and of salts thereof according to Claim 1, characterized in that, in formula (I),
$R^1$ represents hydrogen, or represents $C_1$-$C_4$-alkyl which is optionally substituted by fluorine, cyano, methoxy or ethoxy, or represents allyl, or represents $C_3$-$C_6$-cycloalkyl, or represents phenyl, or represents $C_1$-$C_4$-alkoxy, $C_3$-$C_4$-alkenyloxy, or represents $C_1$-$C_3$-alkylamino, $C_3$-$C_6$-cycloalkylamino or represents di-($C_1$-$C_3$-alkyl)-amino,
$R^2$ represents chlorine or bromine and
$R^3$ represents the grouping

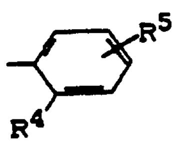

wherein
$R^4$ represents fluorine, chlorine, bromine, methyl, trifluoromethyl, methoxy, difluoromethoxy, trifluoromethoxy, 2-chloroethoxy, 2-methoxyethoxy, $C_1$-$C_3$-alkylthio, $C_1$-$C_3$-alkylsulphinyl, $C_1$-$C_3$-alkyl-sulphonyl, dimethylaminosulphonyl, diethylaminosulphonyl, N-methoxy-N-methylaminosulphonyl, N-methoxyaminosulphonyl, phenyl, phenoxy or $C_1$-$C_3$-alkoxy-carbonyl and
$R^5$ represents hydrogen, fluorine, chlorine or bromine;
or furthermore
$R^3$ represents the radical

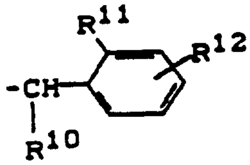

wherein

$R^{10}$ represents hydrogen,
$R^{11}$ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
$R^{12}$ represents hydrogen;

or furthermore
$R^3$ represents the radical

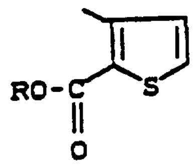

wherein R represents $C_1$-$C_4$-alkyl, or
represents the radical

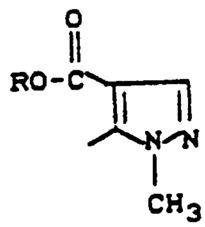

wherein R represents $C_1$-$C_4$-alkyl.

**3.** Herbicidal compositions, characterized in that they contain at least one halogenated sulphonylaminocarbonyltriazolinone of the formula (I) according to Claim 1.

**4.** Use of halogenated sulphonylaminocarbonyltriazolinones of the general formula (I) according to Claim 1 for combating undesirable plant growth.

**5.** Method of combating weeds, characterized in that halogenated sulphonylaminocarbonyltriazolinones of the general formula (I) according to Claim 1 are allowed to act on the weeds or their environment.

**6.** Process for the preparation of herbicidal compositions, characterized in that halogenated sulphonylaminocarbonyltriazolinones of the general formula (I) according to Claim 1 are mixed with extenders and/or surface-active agents.

**7.** Process for the preparation of halogenated triazolinones of the formula (IV)

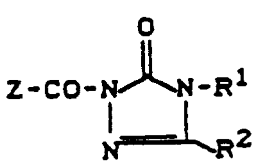

(IV)

in which
$R^1$ and $R^2$ have the meanings given in Claim 1 and
Z represents chlorine, $C_1$-$C_4$-alkoxy, benzyloxy or phenoxy,

characterized in that halogenated triazolinones of the formula (II)

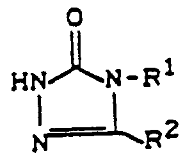

(II)

in which
$R^1$ and $R^2$ have the abovementioned meanings,
are reacted with phosgene, methyl chloroformate, benzyl chloroformate, phenyl chloroformate or diphenyl carbonate.

**Revendications**

**Revendications pour les Etats contractants suivants : BE, CH, DE, DK, FR, GB, IT, LI, NL**

**1.** Sulfonylaminocarbonyltriazolinones halogénées répondant à la formule générale (I)

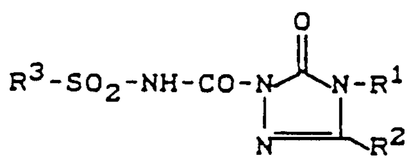

(I)

dans laquelle

$R^1$ représente un atome d'hydrogène, un groupe hydroxyle, un groupe amino; un groupe alkyle en $C_1$-$C_6$ portant éventuellement un ou plusieurs substituants identiques ou différents fluoro, chloro, bromo, cyano, alcoxy en $C_1$-$C_4$, alkyl(en $C_1$-$C_4$)-carbonyle ou alcoxy(en $C_1$- $C_4$)carbonyle; un groupe alcényle en $C_3$-$C_6$ ou un groupe alcynyle en $C_3$-$C_6$ portant respectivement éventuellement un ou plusieurs substituants identiques ou différents fluoro, chloro et/ou bromo; un groupe cycloalkyle en $C_3$-$C_6$ portant éventuellement un ou plusieurs substituants identiques ou différents fluoro, chloro, bromo et/ou alkyle en $C_1$-$C_4$; un groupe phénylalkyle en $C_1$-$C_3$ portant éventuellement un ou plusieurs substituants identiques ou différents fluoro, chloro, bromo, cyano, nitro, alkyle en $C_1$-$C_4$, trifluorométhyle, alcoxy en $C_1$-$C_4$ et/ou alcoxy(en $C_1$-$C_4$)carbonyle; un groupe phényle portant éventuellement un ou plusieurs substituants identiques ou différents fluoro, chloro, bromo, cyano, nitro, alkyle en $C_1$-$C_4$, trifluorométhyle, alcoxy en $C_1$-$C_4$, alcoxy en $C_1$-$C_3$ portant un ou plusieurs substituants identiques ou différents fluoro et/ou chloro, alkyl(en $C_1$-$C_4$)thio, alkyl(en $C_1$-$C_3$)thio portant un ou plusieurs substituants identiques ou différents fluoro et/ou chloro, alkyl(en $C_1$-$C_4$)sulfinyle, alkyl(en $C_1$-$C_4$)sulfonyle et/ou alcoxy(en $C_1$- $C_4$)carbonyle; un groupe alcoxy en $C_1$-$C_6$ portant éventuellement un ou plusieurs substituants identiques ou différents fluoro, chloro, cyano, alcoxy en $C_1$-$C_4$ ou alcoxy(en $C_1$-$C_4$)carbonyle; un groupe alcényl(en $C_3$-$C_4$)oxy; un groupe alkyl(en $C_1$-$C_4$)amino portant éventuellement un ou plusieurs substituants identiques ou différents fluoro, cyano, phényle, alcoxy en $C_1$-$C_4$ ou alcoxy(en $C_1$-$C_4$)carbonyle; un groupe cycloalkyl(en $C_3$-$C_6$)amino ou encore un groupe di-(alkyl en $C_1$-$C_4$)amino,

$R^2$ représente un atome de fluor, un atome de chlore, un atome de brome ou un atome d'iode, et

$R^3$ représente le groupement

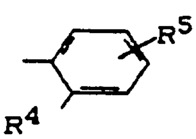

dans lequel

$R_4$ et $R_5$ sont identiques ou différents et représentent un atome d'hydrogène, un atome de fluor, un atome de chlore, un atome de brome, un atome d'iode, un groupe nitro, un groupe alkyle en $C_1$-$C_6$ (qui porte éventuellement un ou plusieurs substituants identiques ou différents fluoro, chloro, bromo, cyano, carboxyle, alcoxy(en $C_1$-$C_4$)-carbonyle, alkyl(en $C_1$-$C_4$)aminocarbonyle, di-(alkyl en $C_1$-$C_4$)aminocarbonyle, hydroxyle, alcoxy en $C_1$-$C_4$, formyloxy, alkyl(en $C_1$-$C_4$)carbonyloxy, alcoxy(en $C_1$-$C_4$)-carbonyloxy, alkyl(en $C_1$-$C_4$)aminocarbonyloxy, alkyl(en $C_1$-$C_4$)thio, alkyl(en $C_1$– $C_4$)-sulfinyle, alkyl(en $C_1$-$C_4$)sulfonyle, di-(alkyl en $C_1$-$C_4$)aminosulfonyle, cycloalkyle en $C_3$-$C_6$ ou phényle); un groupe alcényle en $C_2$-$C_6$ (qui porte éventuellement un ou plusieurs substituants identiques ou différents fluoro, chloro, bromo, cyano, alcoxy-(en $C_1$-$C_4$)carbonyle, carboxyle ou phényle); un groupe alcynyle en $C_2$-$C_6$ (qui porte éventuellement un ou plusieurs substituants fluoro, chloro, bromo, cyano, alcoxy(en $C_1$-$C_4$) carbonyle, carboxyle ou phényle); un groupe alcoxy en $C_1$-$C_4$ (qui porte éventuellement un ou plusieurs substituants identiques ou différents fluoro, chloro, bromo, cyano, carboxyle, alcoxy(en $C_1$-$C_4$)carbonyle, alcoxy en $C_1$-$C_4$, alkyl(en $C_1$-$C_4$)thio, alkyl(en $C_1$-$C_4$)sulfinyle ou alkyl(en $C_1$-$C_4$)sulfonyle); un groupe alkyl(en $C_1$-$C_4$)thio (qui porte éventuellement un ou plusieurs substituants identiques ou différents fluoro, chloro, bromo, cyano, carboxyle, alcoxy(en $C_1$-$C_4$)carbonyle, alkyl(en $C_1$-$C_4$)thio, alkyl(en $C_1$-$C_4$)sulfinyle ou alkyl(en $C_1$-$C_4$)sulfonyle); un groupe alcényl-(en $C_3$-$C_6$)oxy (qui porte éventuellement un ou plusieurs substituants identiques ou différents fluoro, chloro, bromo, cyano ou alcoxy(en $C_1$-$C_4$)carbonyle); un groupe alcényl(en $C_2$-$C_6$)thio (qui porte éventuellement un ou plusieurs substituants identiques ou différents fluoro, chloro, bromo, cyano, nitro, alkyl(en $C_1$-$C_3$)thio ou alcoxy-(en $C_1$-$C_4$)carbonyle); un groupe alcynyl(en $C_3$-$C_6$)oxy, un groupe alcynyl(en $C_3$-$C_6$)-thio, ou encore le radical $-S(O)_p-R^6$ où

p représente les nombres 1 ou 2, et

$R^6$ représente un groupe alkyle en $C_1$-$C_4$ (qui porte éventuellement un ou plusieurs substituants identiques ou différents fluoro, chloro, bromo, cyano ou alcoxy(en $C_1$-$C_4$)carbonyle); un groupe alcényle en $C_3$-$C_6$, un groupe alcynyle en $C_3$-$C_6$, un groupe alcoxy en $C_1$-$C_4$, un groupe alcoxy (en $C_1$-$C_4$)alkyl(en $C_1$-$C_4$)amino, un groupe alkyl(en $C_1$-$C_4$)amino, un groupe di-(alkyl en $C_1$-$C_4$) amino, un groupe phényle ou encore le radical $-NHOR^7$ où

$R^7$ représente un groupe alkyle en $C_1$-$C_{12}$ (qui porte éventuellement un ou plusieurs substituants identiques ou différents fluoro, chloro, cyano, alcoxy en $C_1$-$C_4$, alkyl(en $C_1$-$C_4$)thio, alkyl(en $C_1$-$C_4$)sulfinyle, alkyl(en $C_1$-$C_4$)sulfonyle, alkyl(en $C_1$-$C_4$)-carbonyle, alcoxy(en $C_1$-$C_4$)carbonyle, alkyl(en $C_1$– $C_4$)aminocarbonyle ou di-(alkyl en $C_1$-$C_4$) aminocarbonyle); un groupe alcényle en $C_3$-$C_6$ (qui porte éventuellement un ou plusieurs substituants identiques ou différents fluoro, chloro ou bromo); un groupe alcynyle en $C_3$-$C_6$, un groupe cycloalkyle en $C_3$-$C_6$, un groupe cycloalkyl(en $C_3$-$C_6$)alkyle en $C_1$-$C_2$; un groupe phénylalkyle en $C_1$-$C_2$ (qui porte éventuellement un ou plusieurs substituants identiques ou différents fluoro, chloro, nitro, cyano, alkyle en $C_1$-$C_4$, alcoxy en $C_1$-$C_4$ ou alcoxy(en $C_1$– $C_4$)carbonyle); un groupe benzhydryle; ou encore un groupe phényle (qui porte éventuellement un ou plusieurs substituants identiques ou différents fluoro, chloro, nitro, cyano, alkyle en $C_1$-$C_4$, trifluorométhyle, alcoxy en $C_1$-$C_4$, fluoralcoxy en $C_1$-$C_2$, alkyl(en $C_1$-$C_4$)thio, trifluorométhylthio ou alcoxy(en $C_1$-$C_4$)carbonyle),

$R^4$ et/ou $R^5$ représentent, en outre, un groupe phényle ou un groupe phénoxy; un groupe alkyl-(en $C_1$-$C_4$)carbonylamino, un groupe alcoxy(en $C_1$-$C_4$)carbonylamino, un groupe alkyl(en $C_1$-$C_4$)aminocarbonylamino, un groupe di-(alkyl en $C_1$-$C_4$)-aminocarbonylamino ou encore le radical $-CO-R^8$ où

$R^8$ représente un groupe alkyle en $C_1$-$C_6$, un groupe alcoxy en $C_1$-$C_6$, un groupe cycloalcoxy en $C_3$-$C_6$, un groupe alcényl(en $C_3$-$C_6$)oxy, un groupe alkyl(en $C_1$-$C_4$)-thio, un groupe alkyl(en $C_1$-$C_4$)amino, un groupe alcoxy(en $C_1$– $C_4$)amino, un groupe alcoxy(en $C_1$-$C_4$)alkyl(en $C_1$-$C_4$)amino ou encore un groupe di-(alkyl en $C_1$-$C_4$)amino (qui portent éventuellement un ou plusieurs substituants identiques ou différents fluoro et/ou chloro),

$R^4$ et/ou $R^5$ représentent, en outre, un groupe triméthylsilyle, un groupe thiazolinyle, un groupe

alkyl(en $C_1$-$C_4$)sulfonyloxy, un groupe di-(alkyl en $C_1$-$C_4$)aminosulfonylamino ou encore le radical -CH = N-$R^9$ où

$R^9$ représente un groupe alkyle en $C_1$-$C_6$ portant éventuellement un ou plusieurs substituants identiques ou différents fluoro, chloro, cyano, carboxyle, alcoxy en $C_1$-$C_4$, alkyl(en $C_1$-$C_4$)thio, alkyl(en $C_1$-$C_4$)sulfinyle ou alkyl(en $C_1$-$C_4$)sulfonyle; un groupe benzyle portant éventuellement un ou plusieurs substituants identiques ou différents fluoro ou chloro; un groupe alcényle en $C_3$-$C_6$ ou un groupe alcynyle en $C_3$-$C_6$ portant éventuellement un ou plusieurs substituants identiques ou différents fluoro ou chloro; un groupe phényle portant éventuellement un ou plusieurs substituants identiques ou différents fluoro, chloro, bromo, alkyle en $C_1$-$C_4$, alcoxy en $C_1$-$C_4$, trifluorométhyle, trifluorométhoxy ou trifluorométhylthio; un groupe alcoxy en $C_1$-$C_6$ portant éventuellement un ou plusieurs substituants identiques ou différents fluoro et/ou chloro; un groupe alcénoxy en $C_3$-$C_6$, un groupe alcynoxy en $C_3$-$C_6$ ou encore un groupe benzyloxy; un groupe amino, un groupe alkyl(en $C_1$-$C_4$)amino, un groupe di-(alkyl en $C_1$-$C_4$)amino, un groupe phénylamino, un groupe alkyl(en $C_1$-$C_4$)carbonylamino, un groupe alcoxy(en $C_1$- $C_4$)carbonylamino, un groupe alkyl(en $C_1$- $C_4$)sulfonylamino; ou encore un groupe phénylsulfonylamino portant éventuellement un ou plusieurs substituants identiques ou différents fluoro, chloro, bromo ou méthyle;

dans laquelle, en outre,

$R^3$ représente le radical

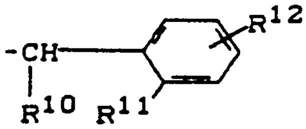

où

$R^{10}$ représente un atome d'hydrogène ou un groupe alkyle en $C_1$-$C_4$,

$R^{11}$ et $R^{12}$ sont identiques ou différents et représentent un atome d'hydrogène, un atome de fluor, un atome de chlore, un atome de brome, un groupe nitro, un groupe cyano, un groupe alkyle en $C_1$-$C_4$ (qui porte éventuellement un ou plusieurs substituants identiques ou différents fluoro et/ou chloro), un groupe alcoxy en $C_1$-$C_4$ (qui porte éventuellement un ou plusieurs substituants identiques ou différents fluoro et/ou chloro), un groupe carboxyle, un groupe alcoxy(en $C_1$- $C_4$)carbonyle, un groupe diméthylaminocarbonyle, un groupe alkyl(en $C_1$-$C_4$)sulfonyle ou encore un groupe di-(alkyl en $C_1$-$C_4$)aminosulfonyle;

dans laquelle, en outre,

$R^3$ représente le radical

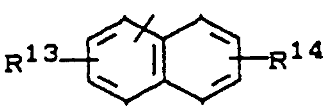

où

$R^{13}$ et $R^{14}$ sont identiques ou différents et représentent un atome d'hydrogène, un atome de fluor, un atome de chlore, un atome de brome, un groupe nitro, un groupe cyano, un groupe alkyle en $C_1$-$C_4$ (qui porte éventuellement un ou plusieurs substituants identiques ou différents fluoro et/ou chloro) ou encore un groupe alcoxy en $C_1$-$C_4$ (qui porte éventuellement un ou plusieurs substituants identiques ou différents fluoro et/ou chloro);

dans laquelle, en outre,

$R^3$ représente le radical

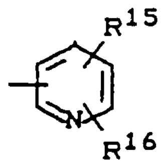

où

$R^{15}$ et $R^{16}$ sont identiques ou différents et représentent un atome d'hydrogène, un atome de fluor, un atome de chlore, un atome de brome, un groupe nitro, un groupe cyano, un groupe alkyle en $C_1$-$C_4$ (qui porte éventuellement un ou plusieurs substituants identiques ou différents fluoro et/ou chloro), un groupe alcoxy en $C_1$-$C_4$ (qui porte éventuellement un ou plusieurs substituants identiques ou différents fluoro et/ou chloro); un groupe alkyl(en $C_1$-$C_4$)thio, un groupe alkyl(en $C_1$-$C_4$)sulfinyle ou un groupe alkyl(en $C_1$-$C_4$)sulfonyle (qui portent éventuellement un ou plusieurs substituants identiques ou différents fluoro et/ou chloro); un groupe aminosulfonyle, un groupe mono-(alkyl en $C_1$-$C_4$)aminosulfonyle, un groupe di-(alkyl en $C_1$-$C_4$)-aminosulfonyle ou un groupe alcoxy(en $C_1$-$C_4$)carbonyle ou encore un groupe diméthylaminocarbonyle;

dans laquelle, en outre,

$R^3$ représente le radical

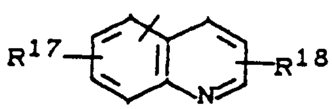

où

$R^{17}$ et $R^{18}$ sont identiques ou différents et représentent un atome d'hydrogène, un atome de fluor, un atome de chlore, un atome de brome, un groupe alkyle en $C_1$-$C_4$ (qui porte éventuellement un ou plusieurs substituants identiques ou différents fluoro et/ou bromo), un groupe alcoxy en $C_1$-$C_4$ (qui porte éventuellement un ou plusieurs substituants identiques ou différents fluoro et/ou chloro); un groupe alkyl(en $C_1$-$C_4$)-thio, un groupe alkyl(en $C_1$-$C_4$)sulfinyle ou un groupe alkyl(en $C_1$-$C_4$)sulfonyle (qui portent éventuellement un ou plusieurs substituants identiques ou différents fluoro et/ou chloro); ou encore un groupe di-(alkyl en $C_1$-$C_4$)aminosulfonyle;

dans laquelle, en outre,

$R^3$ représente le radical

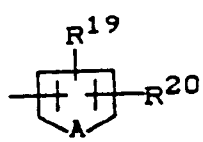

où

$R^{19}$ et $R^{20}$ sont identiques ou différents et représentent un atome d'hydrogène, un atome de fluor, un atome de chlore, un atome de brome, un groupe cyano, un groupe nitro, un groupe alkyle en $C_1$-$C_4$ (qui porte éventuellement un ou plusieurs substituants identiques ou différents fluoro et/ou chloro); un groupe alcoxy en $C_1$-$C_4$ (qui porte éventuellement un ou plusieurs substituants identiques ou différents fluoro et/ou chloro); un groupe alkyl(en $C_1$-$C_4$)thio, un groupe alkyl(en $C_1$-$C_4$)sulfinyle ou un

groupe alkyl(en $C_1$-$C_4$)sulfonyle (qui portent éventuellement un ou plusieurs substituants identiques ou différents fluoro et/ou chloro); un groupe di-(alkyl en $C_1$- $C_4$)-aminosulfonyle, un groupe alcoxy(en $C_1$- $C_4$)carbonyle ou encore un groupe diméthylaminocarbonyle; et

A représente un atome d'oxygène, un atome de soufre ou encore le groupement N-$Z^1$ où

$Z^1$ représente un atome d'hydrogène, un groupe alkyle en $C_1$-$C_4$ (qui porte éventuellement un ou plusieurs substituants identiques ou différents fluoro, chloro, bromo ou cyano); un groupe cycloalkyle en $C_3$-$C_6$, un groupe benzyle, un groupe phényle (qui porte éventuellement un ou plusieurs substituants identiques ou différents fluoro, chloro, bromo et/ou nitro); un groupe alkyl(en $C_1$- $C_4$)carbonyle, un groupe alcoxy(en $C_1$- $C_4$)carbonyle ou encore un groupe di-(alkyl en $C_1$-$C_4$)aminocarbonyle;

dans laquelle, en outre,

$R^3$ représente le radical

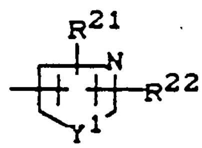

où

$R^{21}$ et $R^{22}$ sont identiques ou différents et représentent un atome d'hydrogène, un groupe alkyle en $C_1$-$C_4$, un atome d'halogène, un groupe alcoxy(en $C_1$-$C_4$)carbonyle, un groupe alcoxy en $C_1$-$C_4$ ou encore un groupe halogénalcoxy en $C_1$-$C_4$,

$Y^1$ représente un atome de soufre ou encore le groupement N-$R^{23}$ où

$R^{23}$ représente un atome d'hydrogène ou un groupe alkyle en $C_1$-$C_4$;

dans laquelle, en outre,

$R^3$ représente le radical

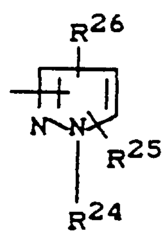

où

$R^{24}$ représente un atome d'hydrogène, un groupe alkyle en $C_1$-$C_4$, un groupe benzyle, un groupe pyridyle, un groupe quinoléinyle ou un groupe phényle,

$R^{25}$ représente un atome d'hydrogène, un atome d'halogène, un groupe cyano, un groupe nitro, un groupe alkyle en $C_1$-$C_4$ (qui porte éventuellement un ou plusieurs substituants identiques ou différents fluoro et/ou chloro); un groupe alcoxy en $C_1$-$C_4$ (qui porte éventuellement un ou plusieurs substituants identiques ou différents fluoro et/ou chloro); un groupe dioxolanyle ou un groupe alcoxy(en $C_1$-$C_4$)carbonyle, et

$R^{26}$ représente un atome d'hydrogène, un atome d'halogène ou un groupe alkyle en $C_1$-$C_4$;

dans laquelle, en outre,

$R^3$ représente un des groupements indiqués ci-après

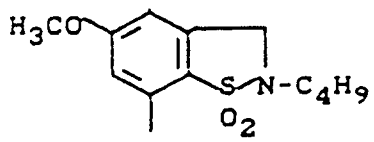 ; 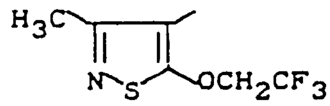

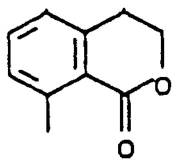

ainsi que des sels des composés de formule (I).

2. Sulfonylaminocarbonyltriazolinones halogénées répondant à la formule générale (I) selon la revendication 1, dans laquelle

$R^1$ représente un atome d'hydrogène; un groupe alkyle en $C_1$-$C_4$ portant éventuellement un ou plusieurs substituants identiques ou différents fluoro, cyano, méthoxy ou éthoxy; un groupe allyle; un groupe cycloalkyle en $C_3$-$C_6$; un groupe phényle; un groupe alcoxy en $C_1$-$C_4$; un groupe alcényl(en $C_3$-$C_4$)oxy; un groupe alkyl(en $C_1$-$C_3$)amino, un groupe cycloalkyl(en $C_3$-$C_6$)amino ou encore un groupe di-(alkyl en $C_1$-$C_3$)amino,

$R^2$ représente un atome de chlore ou un atome de brome, et

$R^3$ représente le groupement

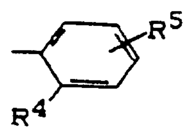

dans lequel

$R^4$ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloréthoxy, un groupe 2-méthoxyéthoxy, un groupe alkyl(en $C_1$-$C_3$)thio, un groupe alkyl(en $C_1$-$C_3$)sulfinyle, un groupe alkyl(en $C_1$- $C_3$)sulfonyle, un groupe diméthylaminosulfonyle, un groupe diéthylaminosulfonyle, un groupe N-méthoxy-N-méthylaminosulfonyle, un groupe N-méthoxyaminosulfonyle, un groupe phényle, un groupe phénoxy ou encore un groupe alcoxy(en $C_1$- $C_3$)carbonyle, et

$R^5$ représente un atome d'hydrogène, un atome de fluor, un atome de chlore ou un atome de brome;

dans laquelle, en outre,

$R^3$ représente le radical

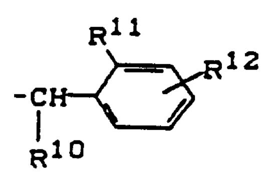

dans lequel

$R^{10}$ représente un atome d'hydrogène,

$R^{11}$ représente un atome de fluor, un atome de chlore, un atome de brome, un groupe méthyle, un groupe méthoxy, un groupe difluorométhoxy, un groupe trifluorométhoxy, un groupe

éthoxy, un groupe méthoxycarbonyle, un groupe éthoxycarbonyle, un groupe méthylsulfonyle ou un groupe diméthylaminosulfonyle, et

$R^{12}$ représente un atome d'hydrogène;

dans laquelle, en outre,

$R^3$ représente le radical

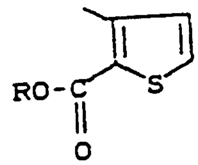

où R représente un groupe alkyle en $C_1$-$C_4$ ou encore représente le radical

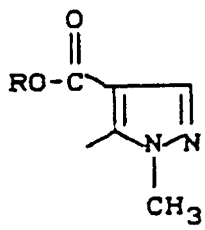

où R représente un groupe alkyle en $C_1$-$C_4$,

ainsi que des sels des composés de formule (I).

**3.** Procédé pour la préparation de sulfonylaminocarbonyltriazolinones halogénées répondant à la formule générale (I) selon la revendication 1, ainsi que des sels des composés de formule (I), caractérisé en ce qu'on fait réagir

a) des triazolinones halogénées répondant à la formule générale (II)

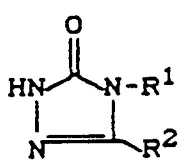

(II)

dans laquelle

$R^1$ et $R^2$ ont les significations indiquées à la revendication 1,

avec des sulfonylisocyanates répondant à la formule générale (III)

$R^3$-$SO_2$-N = C = O (III)

dans laquelle

$R^3$ a la signification indiquée à la revendication 1,

éventuellement en présence d'un diluant, ou bien en ce qu'on fait réagir

b) des dérivés de triazolinone halogénés répondant à la formule générale (IV)

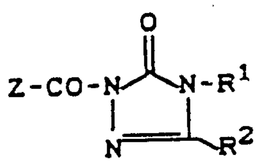

(IV)

dans laquelle
$R^1$ et $R^2$ ont les significations indiquées à la revendication 1, et
Z représente un atome de chlore, un groupe alcoxy en $C_1$-$C_4$, un groupe benzyloxy ou un groupe phénoxy,
avec des amides d'acides sulfoniques répondant à la formule générale (V)

$R^3$-$SO_2$-$NH_2$ (V)

dans laquelle
$R^3$ a la signification indiquée dans la revendication 1,
éventuellement en présence d'un accepteur d'acides et éventuellement en présence d'un diluant, ou bien en ce qu'on fait réagir
c) des triazolinones halogénées répondant à la formule générale (II)

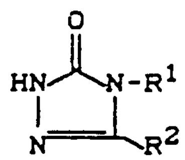

(II)

dans laquelle
$R^1$ et $R^2$ ont les significations indiquées à la revendication 1,
avec des dérivés d'amides d'acides sulfoniques répondant à la formule générale (VI)

$R^3$-$SO_2$-NH-CO-Z (VI)

dans laquelle
$R^3$ a la signification indiquée à la revendication 1, et
Z représente un atome de chlore, un groupe alcoxy en $C_1$-$C_4$, un groupe benzyloxy ou un groupe phénoxy,
éventuellement en présence d'un accepteur d'acides et éventuellement en présence d'un diluant, et, le cas échéant, à partir des composés de formule (I) préparés conformément aux procédés (a), (b) ou (c), on obtient des sels conformément à des procédés habituels.

**4.** Agents herbicides caractérisés par une teneur en au moins une sulfonylaminocarbonyltriazolinone halogénée de formule (I) selon la revendication 1.

**5.** Utilisation de sulfonylaminocarbonyltriazolinones halogénées répondant à la formule générale (I) selon la revendication 1, pour lutter contre la croissance non désirée de plantes.

**6.** Procédé pour lutter contre les mauvaises herbes, caractérisé en ce qu'on laisse réagir des sulfonylaminocarbonyltriazolinones halogénées répondant à la formule générale (I) selon la revendication 1, sur les mauvaises herbes ou sur leur biotope.

**7.** Procédé pour la préparation d'agents herbicides, caractérisé en ce qu'on mélange des sulfonylaminocarbonyltriazolinones halogénées répondant à la formule générale (I) selon la revendication 1, avec des

diluants et/ou des agents tensioactifs.

**8.** Triazolinones halogénées répondant à la formule générale (IV)

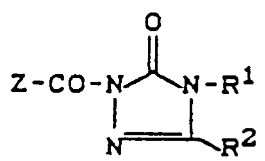

(IV)

dans laquelle

$R^1$ et $R^2$ ont les significations indiquées à la revendication 1,

Z représente un atome de chlore, un groupe alcoxy en $C_1$-$C_4$, un groupe benzyloxy ou un groupe phénoxy.

**Revendications pour l'Etat contractant suivant : ES**

**1.** Procédé pour la préparation de sulfonylaminocarbonyltriazolinones halogénées répondant à la formule générale (I)

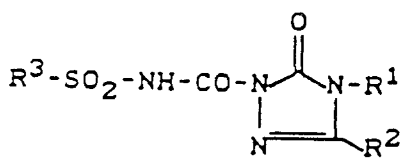

(I)

dans laquelle

$R^1$ représente un atome d'hydrogène, un groupe hydroxyle, un groupe amino; un groupe alkyle en $C_1$-$C_6$ portant éventuellement un ou plusieurs substituants identiques ou différents fluoro, chloro, bromo, cyano, alcoxy en $C_1$-$C_4$, alkyl(en $C_1$-$C_4$)-carbonyle ou alcoxy(en $C_1$- $C_4$)carbonyle; un groupe alcényle en $C_3$-$C_6$ ou un groupe alcynyle en $C_3$-$C_6$ portant respectivement éventuellement un ou plusieurs substituants identiques ou différents fluoro, chloro et/ou bromo; un groupe cycloalkyle en $C_3$-$C_6$ portant éventuellement un ou plusieurs substituants identiques ou différents fluoro, chloro, bromo et/ou alkyle en $C_1$-$C_4$; un groupe phénylalkyle en $C_1$-$C_3$ portant éventuellement un ou plusieurs substituants identiques ou différents fluoro, chloro, bromo, cyano, nitro, alkyle en $C_1$-$C_4$, trifluorométhyle, alcoxy en $C_1$-$C_4$ et/ou alcoxy(en $C_1$-$C_4$)carbonyle; un groupe phényle portant éventuellement un ou plusieurs substituants identiques ou différents fluoro, chloro, bromo, cyano, nitro, alkyle en $C_1$-$C_4$, trifluorométhyle, alcoxy en $C_1$-$C_4$, alcoxy en $C_1$-$C_3$ portant un ou plusieurs substituants identiques ou différents fluoro et/ou chloro, alkyl(en $C_1$-$C_4$)-thio, alkyl(en $C_1$-$C_3$)thio portant un ou plusieurs substituants identiques ou différents fluoro et/ou chloro, alkyl(en $C_1$-$C_4$)sulfinyle, alkyl(en $C_1$-$C_4$)sulfonyle et/ou alcoxy(en $C_1$- $C_4$)carbonyle; un groupe alcoxy en $C_1$-$C_6$ portant éventuellement un ou plusieurs substituants identiques ou différents fluoro, chloro, cyano, alcoxy en $C_1$-$C_4$ ou alcoxy(en $C_1$-$C_4$)carbonyle; un groupe alcényl(en $C_3$-$C_4$)oxy; un groupe alkyl(en $C_1$-$C_4$)amino portant éventuellement un ou plusieurs substituants identiques ou différents fluoro, cyano, phényle, alcoxy en $C_1$-$C_4$ ou alcoxy(en $C_1$-$C_4$)carbonyle; un groupe cycloalkyl(en $C_3$-$C_6$)amino ou encore un groupe di-(alkyl en $C_1$-$C_4$)amino,

$R^2$ représente un atome de fluor, un atome de chlore, un atome de brome ou un atome d'iode, et

$R^3$ représente le groupement

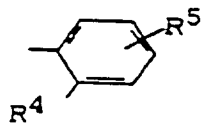

dans lequel

$R_4$ et $R_5$ sont identiques ou différents et représentent un atome d'hydrogène, un atome de fluor, un atome de chlore, un atome de brome, un atome d'iode, un groupe nitro, un groupe alkyle en $C_1$-$C_6$ (qui porte éventuellement un ou plusieurs substituants identiques ou différents fluoro, chloro, bromo, cyano, carboxyle, alcoxy(en $C_1$-$C_4$)-carbonyle, alkyl(en $C_1$-$C_4$)aminocarbonyle, di-(alkyl en $C_1$-$C_4$)aminocarbonyle, hydroxyle, alcoxy en $C_1$-$C_4$, formyloxy, alkyl(en $C_1$-$C_4$)carbonyloxy, alcoxy(en $C_1$-$C_4$)-carbonyloxy, alkyl(en $C_1$-$C_4$)aminocarbonyloxy, alkyl(en $C_1$-$C_4$)thio, alkyl(en $C_1$- $C_4$)-sulfinyle, alkyl(en $C_1$-$C_4$)sulfonyle, di-(alkyl en $C_1$-$C_4$)aminosulfonyle, cycloalkyle en $C_3$-$C_6$ ou phényle); un groupe alcényle en $C_2$-$C_6$ (qui porte éventuellement un ou plusieurs substituants identiques ou différents fluoro, chloro, bromo, cyano, alcoxy-(en $C_1$-$C_4$)carbonyle, carboxyle ou phényle); un groupe alcynyle en $C_2$-$C_6$ (qui porte éventuellement un ou plusieurs substituants fluoro, chloro, bromo, cyano, alcoxy(en $C_1$- $C_4$) carbonyle, carboxyle ou phényle); un groupe alcoxy en $C_1$-$C_4$ (qui porte éventuellement un ou plusieurs substituants identiques ou différents fluoro, chloro, bromo, cyano, carboxyle, alcoxy(en $C_1$-$C_4$)carbonyle, alcoxy en $C_1$-$C_4$, alkyl(en $C_1$-$C_4$)thio, alkyl(en $C_1$-$C_4$)sulfinyle ou alkyl(en $C_1$-$C_4$)sulfonyle); un groupe alkyl(en $C_1$-$C_4$)thio (qui porte éventuellement un ou plusieurs substituants identiques ou différents fluoro, chloro, bromo, cyano, carboxyle, alcoxy(en $C_1$-$C_4$)carbonyle, alkyl(en $C_1$-$C_4$)thio, alkyl(en $C_1$-$C_4$)sulfinyle ou alkyl(en $C_1$-$C_4$) sulfonyle); un groupe alcényl-(en $C_3$-$C_6$)oxy (qui porte éventuellement un ou plusieurs substituants identiques ou différents fluoro, chloro, bromo, cyano ou alcoxy(en $C_1$- $C_4$)carbonyle); un groupe alcényl(en $C_2$-$C_6$)thio (qui porte éventuellement un ou plusieurs substituants identiques ou différents fluoro, chloro, bromo, cyano, nitro, alkyl(en $C_1$-$C_3$)thio ou alcoxy-(en $C_1$-$C_4$)carbonyle); un groupe alcynyl(en $C_3$-$C_6$)oxy, un groupe alcynyl(en $C_3$-$C_6$)-thio, ou encore le radical $-S(O)_p-R^6$ où

p représente les nombres 1 ou 2, et

$R^6$ représente un groupe alkyle en $C_1$-$C_4$ (qui porte éventuellement un ou plusieurs substituants identiques ou différents fluoro, chloro, bromo, cyano ou alcoxy(en $C_1$-$C_4$)carbonyle); un groupe alcényle en $C_3$-$C_6$, un groupe alcynyle en $C_3$-$C_6$, un groupe alcoxy en $C_1$-$C_4$, un groupe alcoxy(en $C_1$-$C_4$)alkyl(en $C_1$-$C_4$)amino, un groupe alkyl(en $C_1$-$C_4$)amino, un groupe di-(alkyl en $C_1$-$C_4$)amino, un groupe phényle ou encore le radical $-NHOR^7$ où

$R^7$ représente un groupe alkyle en $C_1$-$C_{12}$ (qui porte éventuellement un ou plusieurs substituants identiques ou différents fluoro, chloro, cyano, alcoxy en $C_1$-$C_4$, alkyl(en $C_1$-$C_4$)thio, alkyl(en $C_1$-$C_4$)sulfinyle, alkyl(en $C_1$- $C_4$)sulfonyle, alkyl(en $C_1$-$C_4$)-carbonyle, alcoxy(en $C_1$-$C_4$)carbonyle, alkyl(en $C_1$-$C_4$)aminocarbonyle ou di-(alkyl en $C_1$-$C_4$)aminocarbonyle); un groupe alcényle en $C_3$-$C_6$ (qui porte éventuellement un ou plusieurs substituants identiques ou différents fluoro, chloro ou bromo); un groupe alcynyle en $C_3$-$C_6$, un groupe cycloalkyle en $C_3$-$C_6$, un groupe cycloalkyl(en $C_3$-$C_6$)-alkyle en $C_1$-$C_2$; un groupe phénylalkyle en $C_1$-$C_2$ (qui porte éventuellement un ou plusieurs substituants identiques ou différents fluoro, chloro, nitro, cyano, alkyle en $C_1$-$C_4$, alcoxy en $C_1$-$C_4$ ou alcoxy(en $C_1$- $C_4$)carbonyle); un groupe benzhydryle; ou encore un groupe phényle (qui porte eventuellement un ou plusieurs substituants identiques ou différents fluoro, chloro, nitro, cyano, alkyle en $C_1$-$C_4$, trifluorométhyle, alcoxy en $C_1$-$C_4$, fluoralcoxy en $C_1$-$C_2$, alkyl(en $C_1$-$C_4$)thio, trifluorométhylthio ou alcoxy(en $C_1$-$C_4$)carbonyle),

$R^4$ et/ou $R^5$ représentent, en outre, un groupe phényle ou un groupe phénoxy; un groupe alkyl-(en $C_1$-$C_4$)carbonylamino, un groupe alcoxy(en $C_1$-$C_4$)carbonylamino, un groupe alkyl(en $C_1$-$C_4$)aminocarbonylamino, un groupe di-(alkyl en $C_1$-$C_4$)-aminocarbonylamino ou encore le radical $-CO-R^8$ où

$R^8$ représente un groupe alkyle en $C_1$-$C_6$, un groupe alcoxy en $C_1$-$C_6$, un groupe

cycloalcoxy en $C_3$-$C_6$, un groupe alcényl(en $C_3$-$C_6$)oxy, un groupe alkyl(en $C_1$-$C_4$)-thio, un groupe alkyl(en $C_1$-$C_4$)amino, un groupe alcoxy(en $C_1$- $C_4$)amino, un groupe alcoxy(en $C_1$-$C_4$)alkyl(en $C_1$-$C_4$)amino ou encore un groupe di-(alkyl en $C_1$-$C_4$)amino (qui portent éventuellement un ou plusieurs substituants identiques ou différents fluoro et/ou chloro),

$R^4$ et/ou $R^5$ représentent, en outre, un groupe triméthylsilyle, un groupe thiazolinyle, un groupe alkyl(en $C_1$-$C_4$)sulfonyloxy, un groupe di-(alkyl en $C_1$-$C_4$)aminosulfonylamino ou encore le radical -CH=N-$R^9$ où

$R^9$ représente un groupe alkyle en $C_1$-$C_6$ portant éventuellement un ou plusieurs substituants identiques ou différents fluoro, chloro, cyano, carboxyle, alcoxy en $C_1$-$C_4$, alkyl(en $C_1$-$C_4$)thio, alkyl(en $C_1$-$C_4$)sulfinyle ou alkyl(en $C_1$-$C_4$)sulfonyle; un groupe benzyle portant éventuellement un ou plusieurs substituants identiques ou différents fluoro ou chloro; un groupe alcényle en $C_3$-$C_6$ ou un groupe alcynyle en $C_3$-$C_6$ portant éventuellement un ou plusieurs substituants identiques ou différents fluoro ou chloro; un groupe phényle portant éventuellement un ou plusieurs substituants identiques ou différents fluoro, chloro, bromo, alkyle en $C_1$-$C_4$, alcoxy en $C_1$-$C_4$, trifluorométhyle, trifluorométhoxy ou trifluorométhylthio; un groupe alcoxy en $C_1$-$C_6$ portant éventuellement un ou plusieurs substituants identiques ou différents fluoro et/ou chloro; un groupe alcénoxy en $C_3$-$C_6$, un groupe alcynoxy en $C_3$-$C_6$ ou encore un groupe benzyloxy; un groupe amino, un groupe alkyl(en $C_1$-$C_4$)amino, un groupe di-(alkyl en $C_1$-$C_4$)amino, un groupe phénylamino, un groupe alkyl(en $C_1$-$C_4$)carbonylamino, un groupe alcoxy(en $C_1$- $C_4$)carbonylamino, un groupe alkyl(en $C_1$- $C_4$)sulfonylamino; ou encore un groupe phénylsulfonylamino portant éventuellement un ou plusieurs substituants identiques ou différents fluoro, chloro, bromo ou méthyle;

dans laquelle, en outre,

$R^3$ représente le radical

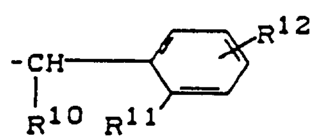

où

$R^{10}$ représente un atome d'hydrogène ou un groupe alkyle en $C_1$-$C_4$,

$R^{11}$ et $R^{12}$ sont identiques ou différents et représentent un atome d'hydrogène, un atome de fluor, un atome de chlore, un atome de brome, un groupe nitro, un groupe cyano, un groupe alkyle en $C_1$-$C_4$ (qui porte éventuellement un ou plusieurs substituants identiques ou différents fluoro et/ou chloro), un groupe alcoxy en $C_1$-$C_4$ (qui porte éventuellement un ou plusieurs substituants identiques ou différents fluoro et/ou chloro), un groupe carboxyle, un groupe alcoxy(en $C_1$- $C_4$)carbonyle, un groupe diméthylaminocarbonyle, un groupe alkyl(en $C_1$-$C_4$)sulfonyle ou encore un groupe di-(alkyl en $C_1$-$C_4$)aminosulfonyle;

dans laquelle, en outre,

$R^3$ représente le radical

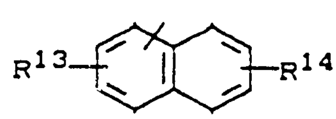

où

$R^{13}$ et $R^{14}$ sont identiques ou différents et représentent un atome d'hydrogène, un atome de fluor, un atome de chlore, un atome de brome, un groupe nitro, un groupe cyano, un

groupe alkyle en $C_1$-$C_4$ (qui porte éventuellement un ou plusieurs substituants identiques ou différents fluoro et/ou chloro) ou encore un groupe alcoxy en $C_1$-$C_4$ (qui porte éventuellement un ou plusieurs substituants identiques ou différents fluoro et/ou chloro);

dans laquelle, en outre,

$R^3$ représente le radical

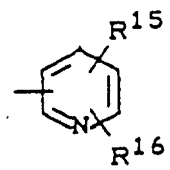

où

$R^{15}$ et $R^{16}$ sont identiques ou différents et représentent un atome d'hydrogène, un atome de fluor, un atome de chlore, un atome de brome, un groupe nitro, un groupe cyano, un groupe alkyle en $C_1$-$C_4$ (qui porte éventuellement un ou plusieurs substituants identiques ou différents fluoro et/ou chloro), un groupe alcoxy en $C_1$-$C_4$ (qui porte éventuellement un ou plusieurs substituants identiques ou différents fluoro et/ou chloro); un groupe alkyl(en $C_1$-$C_4$)thio, un groupe alkyl(en $C_1$-$C_4$)sulfinyle ou un groupe alkyl(en $C_1$-$C_4$)sulfonyle (qui portent éventuellement un ou plusieurs substituants identiques ou différents fluoro et/ou chloro) ; un groupe aminosulfonyle, un groupe mono-(alkyl en $C_1$-$C_4$)aminosulfonyle, un groupe di-(alkyl en $C_1$-$C_4$)-aminosulfonyle ou un groupe alcoxy(en $C_1$-$C_4$)carbonyle ou encore un groupe diméthylaminocarbonyle;

dans laquelle, en outre,

$R^3$ représente le radical

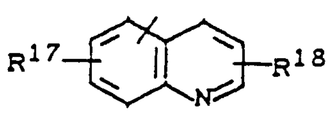

où

$R^{17}$ et $R^{18}$ sont identiques ou différents et représentent un atome d'hydrogène, un atome de fluor, un atome de chlore, un atome de brome, un groupe alkyle en $C_1$-$C_4$ (qui porte éventuellement un ou plusieurs substituants identiques ou différents fluoro et/ou bromo), un groupe alcoxy en $C_1$-$C_4$ (qui porte éventuellement un ou plusieurs substituants identiques ou différents fluoro et/ou chloro); un groupe alkyl(en $C_1$-$C_4$)-thio, un groupe alkyl(en $C_1$-$C_4$)sulfinyle ou un groupe alkyl(en $C_1$-$C_4$)sulfonyle (qui portent éventuellement un ou plusieurs substituants identiques ou différents fluoro et/ou chloro); ou encore un groupe di-(alkyl en $C_1$-$C_4$)aminosulfonyle;

dans laquelle, en outre,

$R^3$ représente le radical

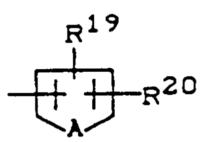

où

$R^{19}$ et $R^{20}$ sont identiques ou différents et représentent un atome d'hydrogène, un atome de

fluor, un atome de chlore, un atome de brome, un groupe cyano, un groupe nitro, un groupe alkyle en $C_1$-$C_4$ (qui porte éventuellement un ou plusieurs substituants identiques ou différents fluoro et/ou chloro); un groupe alcoxy en $C_1$-$C_4$ (qui porte éventuellement un ou plusieurs substituants identiques ou différents fluoro et/ou chloro); un groupe alkyl(en $C_1$-$C_4$)thio, un groupe alkyl(en $C_1$-$C_4$)sulfinyle ou un groupe alkyl(en $C_1$-$C_4$)sulfonyle (qui portent éventuellement un ou plusieurs substituants identiques ou différents fluoro et/ou chloro); un groupe di-(alkyl en $C_1$-$C_4$)-aminosulfonyle, un groupe alcoxy(en $C_1$- $C_4$)carbonyle ou encore un groupe diméthylaminocarbonyle; et

A représente un atome d'oxygène, un atome de soufre ou encore le groupement N-$Z^1$ où

$Z^1$ représente un atome d'hydrogène, un groupe alkyle en $C_1$-$C_4$ (qui porte éventuellement un ou plusieurs substituants identiques ou différents fluoro, chloro, bromo ou cyano); un groupe cycloalkyle en $C_3$-$C_6$, un groupe benzyle, un groupe phényle (qui porte éventuellement un ou plusieurs substituants identiques ou différents fluoro, chloro, bromo et/ou nitro); un groupe alkyl(en $C_1$-$C_4$)carbonyle, un groupe alcoxy(en $C_1$- $C_4$)carbonyle ou encore un groupe di-(alkyl en $C_1$-$C_4$)aminocarbonyle;

dans laquelle, en outre,

$R^3$ représente le radical

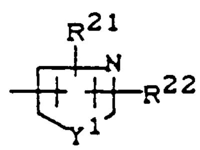

où

$R^{21}$ et $R^{22}$ sont identiques ou différents et représentent un atome d'hydrogène, un groupe alkyle en $C_1$-$C_4$, un atome d'halogène, un groupe alcoxy(en $C_1$-$C_4$)carbonyle, un groupe alcoxy en $C_1$-$C_4$ ou encore un groupe halogénalcoxy en $C_1$-$C_4$,

$Y^1$ représente un atome de soufre ou encore le groupement N-$R^{23}$ où

$R^{23}$ représente un atome d'hydrogène ou un groupe alkyle en $C_1$-$C_4$;

dans laquelle, en outre,

$R^3$ représente le radical

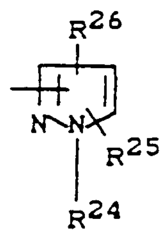

où

$R^{24}$ représente un atome d'hydrogène, un groupe alkyle en $C_1$-$C_4$, un groupe benzyle, un groupe pyridyle, un groupe quinoléinyle ou un groupe phényle,

$R^{25}$ représente un atome d'hydrogène, un atome d'halogène, un groupe cyano, un groupe nitro, un groupe alkyle en $C_1$-$C_4$ (qui porte éventuellement un ou plusieurs substituants identiques ou différents fluoro et/ou chloro); un groupe alcoxy en $C_1$-$C_4$ (qui porte éventuellement un ou plusieurs substituants identiques ou différents fluoro et/ou chloro); un groupe dioxolanyle ou un groupe alcoxy(en $C_1$-$C_4$)carbonyle, et

$R^{26}$ représente un atome d'hydrogène, un atome d'halogène ou un groupe alkyle en $C_1$-$C_4$;

dans laquelle, en outre,

$R^3$ représente un des groupements indiqués ci-après

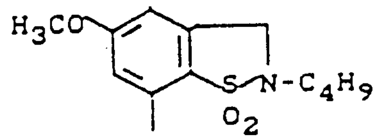 ; 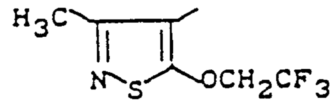

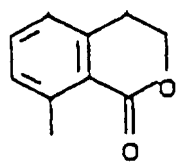

ainsi que de sels des composés de formule (I), caractérisé en ce qu'on fait réagir

a) des triazolinones halogénées répondant à la formule générale (II)

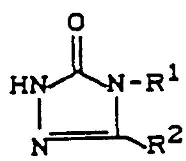

(II)

dans laquelle

$R^1$ et $R^2$ ont les significations indiquées ci-dessus, avec des sulfonylisocyanates répondant à la formule générale (III)

$R^3$-$SO_2$-N = C = O (III)

dans laquelle

$R^3$ a la signification indiquée ci-dessus,

éventuellement en présence d'un diluant, ou bien en ce qu'on fait réagir

b) des dérivés de triazolinone halogénés répondant à la formule générale (IV)

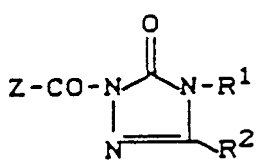

(IV)

dans laquelle

$R^1$ et $R^2$ ont les significations indiquées ci-dessus, et

Z représente un atome de chlore, un groupe alcoxy en $C_1$-$C_4$, un groupe benzyloxy ou un groupe phénoxy,

avec des amides d'acides sulfoniques répondant à la formule générale (V)

$R^3$-$SO_2$-$NH_2$ (V)

dans laquelle

$R^3$ a la signification indiquée ci-dessus,

éventuellement en présence d'un accepteur d'acides et éventuellement en présence d'un diluant, ou bien en ce qu'on fait réagir

c) des triazolinones halogénées répondant à la formule générale (II)

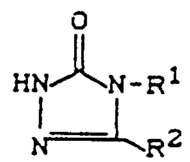

(II)

dans laquelle
$R^1$ et $R^2$ ont les significations indiquées ci-dessus, avec des dérivés d'amides d'acides sulfoniques répondant à la formule générale (VI)

$R^3$-$SO_2$-NH-CO-Z (VI)

dans laquelle
$R^3$ a la signification indiquée ci-dessus, et
Z représente un atome de chlore, un groupe alcoxy en $C_1$-$C_4$, un groupe benzyloxy ou un groupe phénoxy,

éventuellement en présence d'un accepteur d'acides et éventuellement en présence d'un diluant, et, le cas échéant, à partir des composés de formule (I) préparés conformément aux procédés (a), (b) ou (c), on obtient des sels conformément à des procédés habituels.

**2.** Procédé pour la préparation de sulfonylaminocarbonyltriazolinones halogénées de formule (I) et de leurs sels selon la revendication 1, caractérisé en ce que, dans la formule (I),
$R^1$ représente un atome d'hydrogène; un groupe alkyle en $C_1$-$C_4$ portant éventuellement un ou plusieurs substituants identiques ou différents fluoro, cyano, méthoxy ou éthoxy; un groupe allyle; un groupe cycloalkyle en $C_3$-$C_6$; un groupe phényle; un groupe alcoxy en $C_1$-$C_4$; un groupe alcényl(en $C_3$-$C_4$)oxy; un groupe alkyl(en $C_1$-$C_3$)amino, un groupe cycloalkyl(en $C_3$-$C_6$)amino ou encore un groupe di-(alkyl en $C_1$-$C_3$)amino,
$R^2$ représente un atome de chlore ou un atome de brome, et
$R^3$ représente le groupement

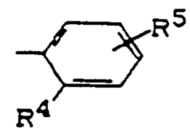

dans lequel
$R^4$ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloréthoxy, un groupe 2-méthoxyéthoxy, un groupe alkyl(en $C_1$-$C_3$)thio, un groupe alkyl(en $C_1$-$C_3$)sulfinyle, un groupe alkyl(en $C_1$- $C_3$)sulfonyle, un groupe diméthylaminosulfonyle, un groupe diéthylaminosulfonyle, un groupe N-méthoxy-N-méthylaminosulfonyle, un groupe N-méthoxyaminosulfonyle, un groupe phényle, un groupe phénoxy ou encore un groupe alcoxy(en $C_1$- $C_3$)carbonyle, et
$R^5$ représente un atome d'hydrogène, un atome de fluor, un atome de chlore ou un atome de brome;

dans laquelle, en outre,
$R^3$ représente le radical

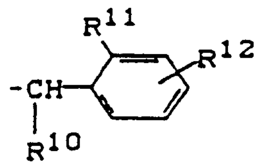

dans lequel

$R^{10}$ représente un atome d'hydrogène,

$R^{11}$ représente un atome de fluor, un atome de chlore, un atome de brome, un groupe méthyle, un groupe méthoxy, un groupe difluorométhoxy, un groupe trifluorométhoxy, un groupe éthoxy, un groupe méthoxycarbonyle, un groupe éthoxycarbonyle, un groupe méthylsulfonyle ou un groupe diméthylaminosulfonyle, et

$R^{12}$ représente un atome d'hydrogène;

dans laquelle, en outre,

$R^3$ représente le radical

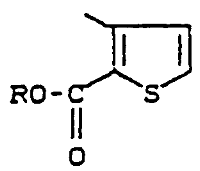

où R représente un groupe alkyle en $C_1$-$C_4$ ou encore représente le radical

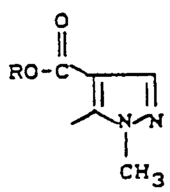

où R représente un groupe alkyle en $C_1$-$C_4$.

3. Agents herbicides caractérisés par une teneur en au moins une sulfonylaminocarbonyltriazolinone halogénée de formule (I) selon la revendication 1.

4. Utilisation de sulfonylaminocarbonyltriazolinones halogénées répondant à la formule générale (I) selon la revendication 1, pour lutter contre la croissance non désirée de plantes.

5. Procédé pour lutter contre les mauvaises herbes, caractérisé en ce qu'on laisse réagir des sulfonylaminocarbonyltriazolinones halogénées répondant à la formule générale (I) selon la revendication 1, sur les mauvaises herbes ou sur leur biotope.

6. Procédé pour la préparation d'agents herbicides, caractérisé en ce qu'on mélange des sulfonylaminocarbonyltriazolinones halogénées répondant à la formule générale (I) selon la revendication 1, avec des diluants et/ou des agents tensioactifs.

**7.** Procédé pour la préparation de triazolinones halogénées de formule (IV)

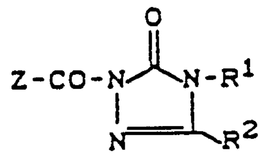

(IV)

dans laquelle

| | |
|---|---|
| $R^1$ et $R^2$ | ont les significations indiquées à la revendication 1, et |
| Z | représente un atome de chlore, un groupe alcoxy en $C_1$-$C_4$, un groupe benzyloxy ou un groupe phénoxy, |

caractérisé en ce qu'on fait réagir des triazolinones halogénées de formule (II)

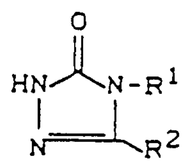

(II)

dans laquelle
$R^1$ et $R^2$ ont les significations indiquées ci-dessus,
avec du phosgène, du chloroformiate de méthyle, du chloroformiate de benzyle, du chloroformiate de phényle ou encore du diphénylcarbonate.